(19) Europäisches Patentamt
European Patent Office
Office européen des brevets

(11) EP 0 820 985 B1

(12) **FASCICULE DE BREVET EUROPEEN**

(45) Date de publication et mention de la délivrance du brevet:
**30.08.2000 Bulletin 2000/35**

(51) Int Cl.[7]: **C07D 209/80**, C07D 519/00, A61K 31/40

(21) Numéro de dépôt: **97401790.7**

(22) Date de dépôt: **24.07.1997**

(54) **Nouveaux dérivés de bis-imides, leur procédé de préparation et les compositions pharmaceutiques qui les contiennent**

Bis-Imide Derivate, Verfahren zu ihrer Herstellung und diese enthaltende pharmazeutische Zubereitung

Bis imide derivatives, process for their preparation and pharmaceutical compositions containing them

(84) Etats contractants désignés:
**AT BE CH DE DK ES FI FR GB GR IE IT LI LU NL PT SE**

(30) Priorité: **26.07.1996 FR 9609417**

(43) Date de publication de la demande:
**28.01.1998 Bulletin 1998/05**

(73) Titulaire: **ADIR ET COMPAGNIE**
**92415 Courbevoie Cédex (FR)**

(72) Inventeurs:
- **Lavielle, Gilbert**
  **78170 La Celle Saint Cloud (FR)**
- **Hautefaye, Patrick**
  **77170 Servon Brie Comte Robert (FR)**
- **Atassi, Ghanem**
  **92210 Saint-Cloud (FR)**
- **Pierre, Alain**
  **78580 Les alluets le roi (FR)**
- **Kraus-Berthier, Laurence**
  **92700 Colombes (FR)**
- **Leonce, Stéphanie**
  **78000 Versailles (FR)**

(56) Documents cités:
**EP-A- 0 506 008** **DE-A- 4 034 687**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux dérivés de bis-imides, leur procédé de préparation, les compositions pharmaceutiques qui les contiennent et leur utilisation pour l'obtention de compositions pharmaceutiques utiles dans le traitement des cancers.

Des composés dérivés de bis-imides ont déjà été décrits dans la littérature. C'est le cas plus particulièrement des composés décrits dans les brevets EP 506 008, DE 4034687, WO 9500490 ou DE 4232739.

Les composés de la présente invention présentent des structures tout à fait originales par rapport à celles précédemment décrites dans l'art antérieur. Ils sont des composés soit symétriques soit disymétriques ce qui n'avait jamais été décrit précédemment. D'autre part, la puissance de leurs activités pharmacologiques les rend particulièrement intéressants comme nouveaux médicaments utiles dans le traitement des cancers et en particulier des tumeurs solides.

Plus spécifiquement, la présente invention concerne les composés de formule (I) :

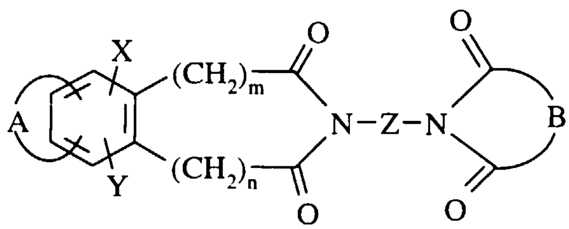

(I)

dans laquelle :

m, n, identiques ou différents, représentent 0 ou 1,

X, Y, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

Z représente une chaîne alkylène $C_4$ à $C_{12}$ linéaire ou ramifié dans laquelle un ou plusieurs groupements $-CH_2-$ sont éventuellement remplacés par l'un quelconque des atomes ou groupements : -NR- (dans lequel R représente un atome d'hydrogène ou un groupement alkyle ($C_1$-$C_6$) linéaire ou ramifié), -O-, -S-, -SO-, $-SO_2-$, -CONH-, ou par un groupement hétérocyclique substitué ou non,

A forme avec deux atomes de carbone adjacents du cycle phényle :

- un cycle phényle substitué ou non,
- un cycle naphtyle substitué ou non,
- un cycle tétrahydronaphtyle substitué ou non, ou 1,4-dioxo-1,2,3,4-tétrahydronapthyle substitué ou non,
- ou, un hétérocycle substitué ou non,

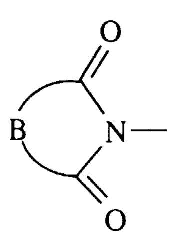

représente l'un quelconque des groupements suivants :

*

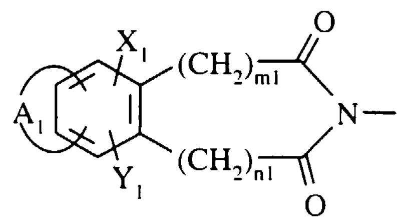

dans lequel :

$m_1$, $n_1$, identiques ou différents, représentent 0 ou 1,

$X_1$, $Y_1$, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

$A_1$ forme avec deux atomes de carbone adjacents du cycle phényle :

- un cycle phényle substitué ou non,
- un cycle naphtyle substitué ou non,
- un cycle tétrahydronaphtyle substitué ou non, ou 1,4-dioxo-1,2,3,4-tétrahydronapthyle substitué ou non,
- ou, un hétérocycle substitué ou non,

*

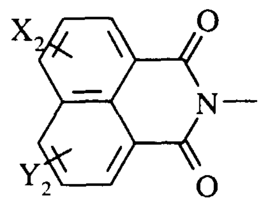

dans lequel $X_2$, $Y_2$, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Par groupement phényle substitué ou non, naphtyle substitué ou non, tétrahydronaphtyle substitué ou non, on entend groupement phényle, napthyle ou tétrahydronaphtyle éventuellement substitué par un ou plusieurs atomes d'halogène ou groupements alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, nitro, cyano ou amino (substitué éventuellement par un ou plusieurs groupements alkyle ($C_1$-$C_6$) linéaire ou ramifié).

Par hétérocycle substitué ou non, on entend un groupement mono ou bicyclique, saturé ou insaturé, de 5 à 16 chaînons contenant un, deux ou trois hétéroatomes choisis parmi oxygène, azote ou soufre, étant entendu que l'hétérocycle peut être éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle, nitro, cyano ou amino (substitué éventuellement par un ou plusieurs groupements alkyle ($C_1$-$C_6$) linéaire ou ramifié).

Les composés préférés selon l'invention sont ceux pour lesquels :

A forme avec deux atomes de carbone adjacents du cycle phényle :

- un cycle naphtyle substitué ou non,

- ou, un hétérocycle substitué ou non préférentiellement choisi parmi les cycles indole éventuellement substitué ou non, benzo[b]thiophène substitué ou non, benzo[b]furane substitué ou non.

Les substituants préférés sont ceux pour lesquels Z représente une chaîne alkylène $C_4$ à $C_{12}$ dans laquelle 1, 2 ou 3 groupements $-CH_2-$ sont remplacés par 1, 2 ou 3 groupements -NR- (dans lequel R représente un groupement alkyle ($C_1$-$C_6$) linéaire ou ramifié).

L'invention s'étend également au procédé de préparation des composés de formule (I) caractérisé en ce que l'on utilise comme produit de départ un anhydride de formule (II) :

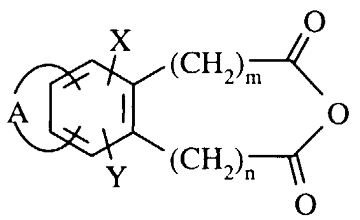

(II)

dans laquelle A, X, Y, m et n sont tels que définis dans la formule (I),
que l'on met en réaction avec un excès d'une diamine de formule (III) :

$$H_2N - Z - NH_2 \qquad (III)$$

dans laquelle Z est tel que défini dans la formule (I),
pour conduire après séparation :

- soit au composé de formule (I/a), cas particulier des composés de formule (I) :

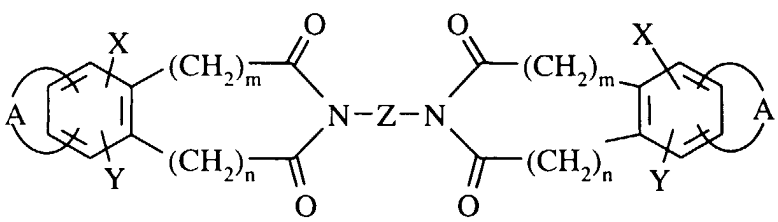

(I/a)

  dans laquelle A, X, Y, Z, m et n ont la même signification que dans la formule (I),
- soit au composé de formule (IV) :

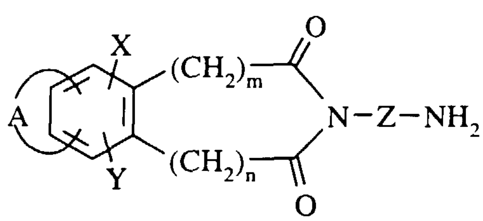

(IV)

  dans laquelle A, X, Y, Z, m et n ont la même signification que dans la formule (I),
- que l'on fait réagir :

  * _soit_ avec l'anhydride de formule (II/a) :

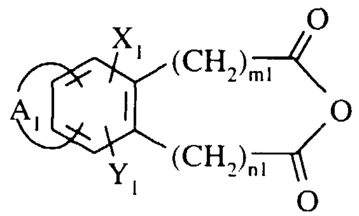

(II/a)

dans laquelle $A_1$, $X_1$, $Y_1$, $m_1$ et $n_1$ sont tels que définis dans la formule (I), pour conduire au composé de formule (I/b), cas particulier des composés de formule (I) :

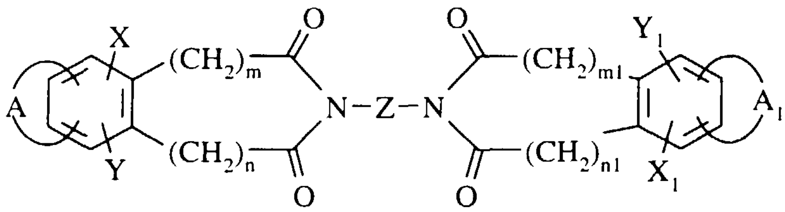

(I/b)

dans laquelle A, X, Y, m, n Z, $A_1$, $X_1$, $Y_1$, $m_1$, $n_1$ ont la même signification que dans la formule (I),

* *soit* avec l'anhydride de formule (V) :

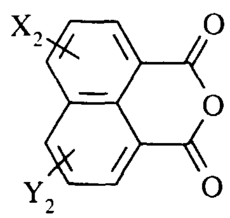

(V)

dans laquelle $X_2$ et $Y_2$ sont tels que définis dans la formule (I),

pour conduire au composé de formule (I/c), cas particulier des composés de formule (I) :

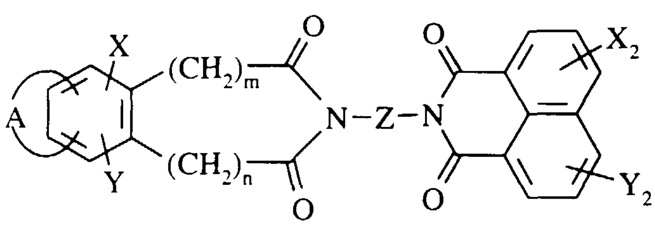

(I/c)

dans laquelle A, X, Y, $X_2$, $Y_2$, Z, m et n ont la même signification que dans la formule (I), composé de formule (I/a), (I/b) ou (I/c), qui peut subir, si on le souhaite, les réactions classiques de transformation des substituants sur les noyaux aromatiques, que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les anhydrides de formule (II) ou (V) sont, soit des composés commerciaux, soit obtenus selon des modes opératoires connus.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration

orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 0,1 à 400 mg par jour en une ou plusieurs prises.

Les exemples suivants illustrent l'invention mais ne la limitent en aucune façon.

Les produits de départ utilisés sont des produits connus ou préparés selon des modes opératoires connus.

Les préparations suivantes conduisent à des intermédiaires de synthèse, utiles pour la préparation des composés de l'invention.

Les structures des composés décrits dans les exemples et les préparations ont été déterminées selon les techniques spectrophotométriques usuelles (infrarouge, RMN, spectrométrie de masse, ...).

**PREPARATION A** : **10-Méthoxy-2-oxa-cyclopenta[c]phénanthrène-1,3-dione**

Stade A : 2-Hydroxyméthylène-7-méthoxy-3,4-dihydro-2H-naphtalèn-1-one

Le produit attendu est préparé à partir de la 7-méthoxy-1-tétralone et du formiate d'éthyle selon le mode opératoire décrit dans Organic synthesis 1959, 39, 27.

Stade B : Ester de tertiobutyle de l'acide 3-(7-méthoxy-1-oxo-3,4-dihydro-1H-napthalèn-2-ylidène)-propionique

On agite 2 heures à température ambiante et sous argon 90 ml d'une solution de 5 mmoles du produit préparé à l'étape précédente et de 6 mmoles d'ester de tertiobutyle de l'acide triphénylphosphanylidène-acétique dans le chlorure de méthylène. Le milieu est concentré sous vide et le résidu est cristallisé dans l'éther isopropylique. Le précipité est filtré, le filtrat est concentré pour fournir 95 % d'un produit jaune qui cristallise.
*Point de fusion : 76°C*

Stade C : 9-Méthoxy-5,6-dihydro-benzo[h]-chromèn-2-one

On ajoute goutte à goutte à température ambiante à 10 ml d'une solution de 4,6 mmoles du produit préparé au stade précédent dans le chlorure de méthylène, 6,5 ml d'anhydride trifluoroacétique, puis 3,6 ml d'acide trifluoroacétique. Le milieu est ensuite agité 4 heures puis hydrolysé à l'aide de 30 ml d'eau. La phase aqueuse est extraite par 30 ml de chlorure de méthylène, la phase organique est lavée à l'eau, séchée et concentrée sous vide.
*Point de fusion : 108-110°C*

Stade D : Ester diméthylique de l'acide 6-méthoxy-9,10-dihydro-phénanthrène-3,4-dicarboxylique

On porte au reflux pendant 4 heures une solution de 5,3 mmoles du produit préparé au stade précédent et de 2,6 ml d'acétylène dicarboxylate de méthyle dans 20 ml de diméthylformamide. Le solvant est évaporé sous vide, le milieu est plongé dans 40 ml d'eau, extrait à l'aide de 3 fois 10 ml d'éther. Les phases organiques réunies sont lavées à l'eau, séchées sur sulfate de magnésium. Après évaporation du solvant, on obtient le produit qui cristallise.
*Point de fusion : 158-160°C*

Stade E : Ester diméthylique de l'acide 6-méthoxy-phénanthrène-3,4-dicarboxylique

Le produit préparé au stade précédent est solubilisé dans 150 ml de toluène sec. On ajoute 1,2 équivalent de dichlorodicyanoquinone et on porte le milieu au reflux pendant 4 heures. Après refroidissement, le milieu est filtré, le filtrat évaporé. Le résidu est alors repris dans 50 ml d'eau et le produit brut est extrait par 3 fois 10 ml d'éther. Après concentration du solvant, le produit est purifié par chromatographie sur silice éluant $CH_2$-$Cl_2$.
*Point de fusion : 112-115°C*

Stade F : Ester de méthyle de l'acide 4-méthoxycarbonyl-6-méthoxyphénanthrène-3-carboxylique et ester de méthyle de l'acide 3-méthoxycarbonyl-6-méthoxyphénanthrène-4-carboxylique

On porte à reflux pendant 3 heures une solution du produit préparé à l'étape précédente dans 15 ml de soude 1 N et 20 ml de méthanol. Après refroidissement, on évapore le méthanol et on acidifie la phase aqueuse à l'aide d'acide chlorhydrique 6 N. Le milieu est extrait par 3 fois 20 ml de chlorure de méthylène et les produits sont obtenus après évaporation du solvant.

Stade G : 10-Méthoxy-2-oxa-cyclopenta[c]phénanthrène-1,3-dione

Un mélange des acides préparés dans l'étape précédente dans 120 ml d'anhydride acétique est porté au reflux 24 h. Après refroidissement, le précipité est filtré, lavé à l'éther et séché.
*Point de fusion : 238°C*

**PREPARATION B : 2-Oxa-cyclopenta[c]phénanthrène-1,3-dione**

Stade A : Ester de diméthyle de l'acide phénanthrène-3,4-dicarboxylique

On porte à reflux pendant 14 heures une solution de 65 mmoles de 2-vinylnaphtalène et 68 mmoles d'acétylène dicarboxylate de méthyle dans 200 ml de nitrobenzène. Après distillation du solvant, le milieu réactionnel est chromatographié sur silice (éluant : $CH_2Cl_2$) pour obtenir le produit attendu.
*Point de fusion : 117°C*

Stade B : Acide 3-méthoxycarbonyl phénanthrène-4-carboxylique et acide 4-méthoxycarbonyl phénanthrène-3-carboxylique

Le mélange est préparé à partir du produit décrit au stade A selon le procédé décrit au stade F de la préparation A.
*Point de fusion : 257°C*

Stade C : 2-Oxa-cyclopenta[c]phénanthrène-1,3-dione

Le produit attendu est préparé à partir du mélange décrit au stade précédent selon le procédé décrit au stade G de la préparation A.
*Point de fusion : 245°C*

**PREPARATION C : Naphto[2,3-c]furane-1,3-dione**

Le produit attendu est obtenu selon le procédé décrit au stade G de la préparation A à partir de l'acide naphtalène-2,3-dicarboxylique.

**PREPARATION D : 2-Oxa-8-thia-dicyclopenta[a,h]naphtalène-1,3-dione**

Le produit attendu est obtenu selon le procédé décrit dans la préparation A en utilisant au stade A le 6,7-dihydro-5H-benzo[b]thiophène-4-one.

**PREPARATION E : 9-Aza-9,10-diméthyl-6-méthoxy-2-oxa-9H-cyclopenta[b]fluorène-1,3-dione**

Stade A : Diester de méthyle de l'acide 1,9-diméthyl-6-méthoxy-9H-carbazole-2,3-dicarboxylique

Le produit attendu est préparé à partir du 1,9-diméthyl-6-méthoxy-9H-pyrano[3,4-b]indol-3-one selon la méthode décrite dans Chem. Ber, 97, 667, 1964.

Stade B : Acide 1,9-diméthyl-6-méthoxy-2-méthoxycarbonyl-9H-carbazole-3-carboxylique et acide 1,9-diméthyl-6-méthoxy-3-méthoxycarbonyl-9H-carbazole-2-carboxylique

Le mélange est préparé à partir du produit décrit au stade A selon le procédé décrit au stade F de la préparation A.

Stade C : 9-Aza-9,10-diméthyl-6-méthoxy-2-oxa-9H-cyclopenta[b]fluorène-1,3-dione

Le produit attendu est obtenu selon le procédé décrit au stade G de la préparation A à partir du composé décrit au stade précédent.

**PREPARATION F : 10-Nitro-2-oxacyclopenta[c]phénanthrène-1,3-dione**

Stade A : Diester de méthyle de l'acide 6-nitro-phénanthrène-3,4-dicarboxylique

Une solution glacée de 38,8 mmoles d'acide nitrique à 70% est ajoutée goutte à goutte à une suspension refroidie à 0°C de 32,3 mmoles du produit obtenu au stade A de la préparation B dans 120 ml d'anhydride trifluoroacétique. Le milieu est agité 3 h à 0°C, puis 2 h à température ambiante. Le précipité est ensuite filtré lavé à l'éther diisopropylique et seché.
*Point de fusion : 180°C avec décomposition*

Stade B : Acide 6-nitro-3-méthoxycarbonyl phénanthrène-4-carboxylique et acide 6-nitro-4-méthoxycarbonyl phénanthrène-3-carboxylique

Le mélange est préparé à partir du produit obtenu dans l'étape précédente selon le procédé décrit au stade B de la préparation G.

Stade C : 10-Nitro-2-oxa-cyclopenta[c]phénanthrène-1,3-dione

Le produit est obtenu selon le procédé décrit au stade G de la préparation A à partir du mélange préparé dans l'étape précédente.

**PREPARATION G : 9-Oxa-cyclopenta[b]phénanthrène-8,10-dione**

Stade A : Ester d'éthyle de l'acide naphtalèn-1-yl-acrylique

A une solution de 0,219 mole de bromure d'éthoxycarbonyl-triphényl-phosphonium dans 200 ml de tétrahydrofurane, sont ajoutés, goutte à goutte, 220 ml d'une solution 1 N de tertiobutylate de potassium dans le tétrahydrofurane. Le milieu est agité 1 h à température ambiante, puis une solution de 0,22 mole de naphtalèn-1-carboxaldéhyde dans le tétrahydrofurane est ajoutée goutte à goutte. Le milieu est agité 4 h à l'ambiante, filtré, le solvant est évaporé, le résidu est repris dans 400 ml de chlorure de méthylène. la phase organique est lavée avec 100 ml d'eau, séchée et concentrée. Le résidu est repris dans de l'ether diisopropylique, le précipité est filtré, le produit attendu est obtenu après concentration du solvant.

Stade B : Acide naphtalèn-1-yl-acrylique

Une solution de 0,2 mole du composé préparé à l'étape précédente dans 500 ml d'éthanol est agitée 18 h à température ambiante en présence de 250 ml d'une solution 2 N d'hydroxyde de sodium . L'éthanol est concentré, le milieu est extrait à l'aide de 100 ml d'acétate d'éthyle, la phase aqueuse est acidifiée à 0°C par une solution d'acide chlorhydrique 6 N. Le produit attendu est filtré et séché.
*Point de fusion : 188°C*

Stade C : Acide 2,3-dibromo-naphtalèn-1-yl-propionique

Le produit attendu est préparé à partir de l'acide obtenu à l'étape précédente selon la méthode décrite dans Aust. J. Chem., 16, 854, 1963.
*Point de fusion : 197°C*

Stade D : Acide naphtalèn-1-yl-propynoïque

On agite à reflux 2 h, puis 16h à l'ambiante un mélange de 0,1 mole du composé préparé à l'étape précédente dans 120 ml d'éthanol contenant 0,36 mole d'hydroxyde de potassium . Le précipité est filtré, le filtrat concentré. Le résidu est repris dans de l'éther, le précipité est filtré, lavé au dichlorométhane, puis dilué dans 400 ml d'eau. La phase aqueuse est acidifiée par de l'acide chlorhydrique concentré, le produit attendu est filtré, séché, recristallisé dans du tétrachlorure de carbone.
*Point de fusion : 95°C*

Stade E : 9-Oxa-cyclopenta[b]phénanthrène-8,10-dione

Le produit attendu est préparé à partir du composé préparé à l'étape précédente selon la méthode décrite dans Aust. J. Chem., 16, 854, 1963.
*Point de fusion : 278°C*

**PREPARATION H : Benzo[d]benzo[2,1-b;3-4-c']difuran-1,3-dione**

Stade A : Ester de méthyle de l'acide benzofuran-3-yl-acétique

Un mélange de 0,4 mole de 3-coumaranone et de 0,48 mole d'ester de méthyle de l'acide (triphénylphosphanylidène)-acétique dans 1 litre de p.xylène est porté au reflux 18 h. Après retour à l'ambiante, le solvant est évaporé, le résidu est repris dans 1 litre d'éther, le précipité est filtré, lavé à l'éther, le filtrat est concentré et le produit attendu est obtenu après chromatographie sur silice (éluant : $CH_2Cl_2$).

Stade B : 2-Benzofuran-3-yl-éthanol

Une solution de 0,38 mole du produit préparé à l'étape précédente est ajoutée à une suspension de 0,64 mole de $AlLiH_4$ dans 1 litre d'éther agitée à 0°C. Le milieu est agité 1 h à l'ambiante avant d'ajouter goutte à goutte 100 ml d'acétate d'éthyle, puis 100 ml d'HCl 1N. Après 18 h d'agitation, le milieu est filtré, le filtrat est décanté, la phase organique est lavée à l'aide d'une solution saturée de NaCl puis à l'eau. Le produit attendu est obtenu après séchage de la phase organique et évaporation du solvant.

Stade C : 3-(2-Bromo-éthyl)-benzofurane

Le produit attendu est obtenu à partir du composé préparé dans l'étape précédente selon le mode opératoire décrit dans Aust. J. Chem., 44, 907, 1991.

Stade D : 3-Vinyl-benzofurane

Le produit attendu est obtenu à partir du composé préparé dans l'étape précédente selon le mode opératoire décrit dans Aust. J. Chem., 44, 907, 1991.

Stade E : Diester de méthyle de l'acide 1,2-dihydro-dibenzofuran-3,4-dicarboxylique

Une solution de 0,2 mole du composé préparé à l'étape précédente et de 0,2 mole d'acétylène dicarboxylate de méthyle dans 1 litre de toluène dégazé est portée au reflux sous atmosphère inerte 24 h. Après concentration du solvant, le produit attendu est obtenu par chromatographie sur silice (éluant : $CH_2Cl_2$).

Stade F : Diester de méthyle de l'acide dibenzofuran-3,4-dicarboxylique

Le produit attendu est obtenu à partir du composé préparé dans l'étape précédente selon le procédé décrit au stade E de la préparation A.
*Point de fusion : 123°C*

Stade G : Acide dibenzofuran-3-4-dicarboxylique

Le produit attendu est obtenu à partir du composé préparé dans l'étape précédente selon le procédé décrit au stade B de la préparation G.
*Point de fusion* : 260-262°C

Stade H : Benzo[d]benzo[2,1-b;3-4-c']difuran-1,3-dione

Le produit attendu est obtenu à partir du composé préparé dans l'étape précédente selon le procédé décrit au stade G de la préparation A.
*Point de fusion : 258°C*

**PREPARATION I : Benzo[d]benzo[1,2-b; 3-4-c']difuran-1,3-dione**

Stade A : 2-Vinyl-benzofurane

A un mélange vivement agité a 0°C de 68,4 mmoles de benzofuran-2-carboxaldéhyde et de 92 mmoles de bromure de méthyl-triphényl-phosphonium dans 200 ml de tétrahydrofurane et 150 ml de diméthylformamide sont ajoutées goutte à goutte 92 ml d'une solution de tertiobutylate de potassium 1 N dans le tétrahydrofurane . Le milieu est agité 2 h à température ambiante, plongé dans 2 litres d'eau glacée, et extrait à l'éther. Après concentration des solvants, le résidu est repris dans 1 litre d'éther de pétrole, le précipité est filtré, lavé à l'éther de pétrole. Le produit attendu est obtenu par évaporation du filtrat.

Stade B : Diester de méthyle de l'acide dibenzofurane-1,2-dicarboxylique

Le produit attendu est obtenu à partir du composé préparé dans l'étape précédente selon le procédé décrit au stade E de la préparation H.
*Point de fusion : 126°C*

Stade C : Acide dibenzofurane-1-méthoxycarbonyl-2-carboxylique et acide dibenzofurane-2-méthoxycarbonyl-1-carboxylique

Le produit attendu est obtenu à partir du composé préparé dans l'étape précédente selon le procédé décrit au stade B de la préparation G.
*Point de fusion : 175-176°C*

Stade D : Benzo[d]benzo[1,2-b; 3-4-c']difuran-1,3-dione

Le produit attendu est obtenu à partir du composé préparé dans l'étape précédente selon le procédé décrit au stade G de la préparation A.
*Point de fusion : 211°C*

**PREPARATION J : 2-Oxa-6-thia-cyclopenta[c]fluorène-1,3-dione**

Stade A : Benzo[b]thiophène-2-carboxaldéhyde

Le produit attendu est obtenu à partir du Benzo[b]thiophène selon la méthode décrite dans J.A.C.S., 74, 2396, 1952.
*Point de fusion : 40-41°C*

Stade B : Vinyl-2-benzo[b]thiophène

Le produit attendu est obtenu à partir du composé préparé dans l'étape précédente selon le procédé décrit au stade A de la préparation I
*Point de fusion : 132-134°C .*

Stade C : Diester de méthyle de l'acide 3,4-dihydro-dibenzothiophène-1,2-dicarboxylique

Le produit attendu est obtenu à partir du composé préparé dans l'étape précédente selon le procédé décrit au stade E de la préparation H.
*Point de fusion : 129°C .*

Stade D : Diester de méthyle de l'acide dibenzothiophène-1,2-dicarboxylique

Le produit attendu est obtenu à partir du composé préparé dans l'étape précédente selon le procédé décrit au stade E de la préparation A.

Stade E : Acide 1-méthoxycarbonyl-dibenzothiophène-2-carboxylique et acide 2-méthoxycarbonyl-dibenzothiophène-1-carboxylique

Le produit attendu est obtenu à partir du composé préparé dans l'étape précédente selon le procédé décrit au stade B de la préparation G.
*Point de fusion : 248-250°C*

Stade F : 2-Oxa-6-thia-cyclopenta[c]fluorène-1,3-dione

Le produit attendu est obtenu à partir du composé préparé dans l'étape précédente selon le procédé décrit au stade G de la préparation A.
*Point de fusion : 269°C*

**PREPARATION K : 10-Méthyl-10*H*-2-oxa-10-aza-cyclopenta[a] fluorène-1,3-dione**

Stade A : 1-Méthyl-3-vinyl-1*H*-indole

Le produit attendu est obtenu à partir du 1-Méthyl-1*H*-indole-3-carboxaldéhyde selon le procédé décrit au stade A de la préparation I.

Stade B : Diester de méthyle de l'acide 9-méthyl-4,9-dihydro-3*H*-carbazole-1,2-dicarboxylique

Le produit attendu est obtenu à partir du composé préparé dans l'étape précédente selon le procédé décrit au stade E de la préparation H.
*Point de fusion : 113°C*

Stade C : Diester de méthyle de l'acide 9-méthyl-3*H*-carbazole-1,2-dicarboxylique

Le produit attendu est obtenu à partir du composé préparé dans l'étape précédente selon le procédé décrit au stade E de la préparation A.
*Point de fusion : 139°C*

Stade D : Acide 9-méthyl-1-methoxycarbonyl-3*H*-indole-2-carboxylique et acide 9-méthyl-2-methoxycarbonyl-3*H*-indole-1-carboxylique

Le produit attendu est obtenu à partir du composé préparé dans l'étape précédente selon le procédé décrit au stade B de la préparation G.

Stade E : 10-Méthyl-10*H*-2-oxa-10-aza-cyclopenta[a]fluorène-1,3-dione

Le produit attendu est obtenu à partir du composé préparé dans l'étape précédente selon le procédé décrit au stade G de la préparation A.
*Point de fusion : >300°C*

**PREPARATION L : 6-Méthyl-*6H*-2-oxa-6-aza-cyclopenta[c]fluorène-1,3-dione**

Stade A : (1-méthyl-1*H*-indol-2-yl)-méthanol

Le produit attendu est obtenu à partir de l'ester de méthyle de l'acide 1-méthyl-1H-indole-2-carboxylique selon le procédé décrit au stade B de la préparation H.
*Point de fusion : 104°C*

Stade B : 1-méthyl-1*H*-indole-2-carboxaldéhyde

Un mélange de 0,1 mole du composé préparé dans l'étape précédente, de 1 mole d'oxyde de manganèse, de 0,37 mole de chlorure de sodium dans 500 ml d'éther est agité 48 h à température ambiante dans une bombe. Le milieu est filtré, concentré, le produit attendu est obtenu par chromatographie sur silice (éluant : $CH_2Cl_2$).
*Point de fusion : 83°C*

Stade C : 1-Méthyl-2-vinyl-1*H*-indole

Le produit attendu est obtenu à partir du composé préparé dans l'étape précédente selon le procédé décrit au stade A de la préparation I.

Stade D : Diester de méthyle de l'acide 9-méthyl-2,9-dihydro-9*H*-carbazole-3,4-dicarboxylique

Le produit attendu est obtenu à partir du composé préparé dans l'étape précédente selon le procédé décrit au stade E de la préparation H.
*Point de fusion : 153°C*

Stade E : Diester de méthyle de l'acide 9-méthyl-9*H*-carbazole-3,4-dicarboxylique

Le produit attendu est obtenu à partir du composé préparé dans l'étape précédente selon le procédé décrit au stade E de la préparation A
*Point de fusion : 156°C*

Stade F : Acide 9-méthyl-9*H*-carbazole-3,4-dicarboxylique

Le produit attendu est obtenu à partir du composé préparé dans l'étape précédente selon le procédé décrit au stade B de la préparation G.
*Point de fusion : 294°C*

Stade G : 6-Méthyl-6*H*-2-oxa-6-aza-cyclopenta[c]fluorène-1,3-dione

Le produit attendu est obtenu à partir du composé préparé dans l'étape précédente selon le procédé décrit au G de la préparation A.
*Point de fusion : >300°C*

**PREPARATION M : 6-Acétyl-6*H*-2-oxa-6-aza-cyclopenta[c]fluorène-1,3-dione**

Stade A : (1*H*-indol-2-yl)-méthanol

Le produit attendu est obtenu à partir de l'ester d'éthyle de l'acide 1H-indole-2-carboxylique selon le procédé décrit au stade B de la préparation H.
*Point de fusion : 80°C*

Stade B : 1*H*-indole-2-carboxaldéhyde

Le produit attendu est obtenu à partir du composé préparé dans l'étape précédente selon le procédé décrit au stade B de la préparation L.

Stade C : 2-Vinyl-1*H*-indole

Le produit attendu est obtenu à partir du composé préparé dans l'étape précédente selon le procédé décrit au stade A de la préparation I.
*Point de fusion : 91°C*

Stade D : Diester de méthyle de l'acide 2,9-dihydro-1*H*-carbazole-3,4-dicarboxylique

Le produit attendu est obtenu à partir du composé préparé dans l'étape précédente selon le procédé décrit au stade E de la préparation H.
*Point de fusion : 249°C*

Stade E : Diester de méthyle de l'acide 1*H*-carbazole-3,4-dicarboxylique

Le produit attendu est obtenu à partir du composé préparé dans l'étape précédente selon le procédé décrit au stade E de la préparation A.

*Point de fusion : 219°C*

Stade F : Acide 3-méthoxycarbonyl-1*H*-carbazole-4-carboxylique et acide 4-méthoxycarbonyl-1*H*-carbazole-3-carboxylique

Le produit attendu est obtenu à partir du composé préparé dans l'étape précédente selon le procédé décrit au stade B de la préparation G.

Stade G : 6-Acétyl-6*H*-2-oxa-6-aza-cyclopenta[c]fluorène-1,3-dione

Le produit attendu est obtenu à partir du composé préparé dans l'étape précédente selon le procédé décrit au stade G de la préparation A.

**PREPARATION N : 6-Méthyl-9-nitro-6*H*-2-oxa-6-aza-cyclopenta[c]fluorène-1,3-dione**

Stade A : Diester de méthyle de l'acide 6-nitro-9-méthyl-9*H*-carbazole-3,4-dicarboxylique

Le produit attendu est obtenu à partir du composé préparé au stade E de la préparation L selon le procédé décrit au stade A de la préparation F.
*Point de fusion : 234°C*

Stade B : Acide 6-nitro-9-méthyl-9*H*-carbazole-3,4-dicarboxylique

Le produit attendu est obtenu à partir du composé préparé dans l'étape précédente selon le procédé décrit au stade F de la préparation A.

Stade C : 6-Méthyl-9-nitro-6*H*-2-oxa-6-aza-cyclopenta[c]fluorène-1,3-dione

Le produit attendu est obtenu à partir du composé préparé dans l'étape précédente selon le procédé décrit au stade G de la préparation A.
*Point de fusion : >300°C*

**PREPARATION O : 6-Méthyl-9-méthoxy-6*H*-2-oxa-6-aza-cyclopenta[c] fluorène-1,3-dione**

Stade A : Ester de méthyle de l'acide 5-méthoxy-1-méthyl-1*H*-indole carboxylique

100 ml d'une solution 1N de tertiobutylate de potassium dans le tétrahydrofurane est ajoutée goutte à goutte à une solution refroidie à -20°C de 0,1 mole d'ester de méthyle de l'acide 5-méthoxy-1*H*-indole carboxylique dans le tétrahydrofurane. Le milieu est agité 30 minutes à cette température avant d'ajouter goutte à goutte une solution de 0,1 mole d'iodure de méthyle dans le tétrahydrofurane. L'addition terminée, le milieu est réchauffé lentement jusqu'a l'ambiante, agité 1h à cette température, filtré. Le produit attendu est obtenu après concentration du solvant.
*Point de fusion : 200°C*

Stade B : (5-méthoxy-1-méthyl-1*H*-indole-2-yl)-méthanol

Le produit attendu est obtenu à partir du composé préparé dans l'étape précédente selon le procédé décrit au stade B de la préparation H.
*Point de fusion : 158°C*

Stade C : 5-méthoxy-1-méthyl-1*H*-indole-2-carboxaldéhyde

Le produit attendu est obtenu à partir du composé préparé dans l'étape précédente selon le procédé décrit au stade B de la préparation L.
*Point de fusion : 88-90°C*

Stade D : 5-Méthoxy-1-méthyl-2-vinyl-1*H*-indole

Le produit attendu est obtenu à partir du composé préparé dans l'étape précédente selon le procédé décrit

au stade A de la préparation I.
*Point de fusion : 87-89°C*

Stade E : Diester de méthyle de l'acide 6-méthoxy-9-méthyl-2,9-dihydro-*1H*-carbazole-3,4-dicarboxylique

Le produit est obtenu à partir du composé préparé au stade précédent selon le procédé décrit au stade E de la préparation H.
*Point de fusion : 245°C*

Stade F : Diester de méthyle de l'acide 6-méthoxy-9-méthyl-*1H*-carbazole-3,4-dicarboxylique

Le produit est obtenu à partir du composé précédent selon le procédé décrit au stade E de la préparation A.
*Point de fusion : 211°C*

Stade G : Acide 6-méthoxy-9-méthyl-*1H*-carbazole-3,4-dicarboxylique

Le produit est obtenu à partir du composé précédent selon le procédé décrit au stade F de la préparation A.

Stade H : 9-Méthoxy-6-méthyl-6H-2-oxa-6-aza-cyclopenta[c]fluorène-1,3-dione

Le produit est obtenu à partir du composé précédent selon le procédé décrit au stade G de la préparation A.

**PREPARATION P : 5,6-Diméthyl-6*H*-2-oxa-6-aza-cyclopenta[c]fluorène-1,3-dione**

Stade A : 2-Isopropenyl-1-méthyl-1*H*-indole

Le produit attendu est obtenu à partir de la N-méthyl-1*H*-indole selon la méthode décrite dans J.O.C., 59, (15), 4250, 1994.

Stade B : Diester de méthyle de l'acide 1,9-diméthyl-1,2-dihydro-9H-carbazole-3,4-dicarboxylique

Le produit attendu est obtenu à partir du produit du stade A selon le procédé décrit au stade E de la préparation H.

Stade C : Diester de méthyle de l'acide 1,9-diméthyl-9H-carbazole-3,4-dicarboxylique.

Le produit attendu est obtenu à partir du produit précédent selon le procédé décrit au stade E de la préparation A.

Stade D : Acide 1,9-diméthyl-9H-carbazole-3,4-dicarboxylique

Le produit attendu est obtenu à partir du composé précédent selon le procédé décrit au stade F de la préparation A.

Stade E : 5,6-Diméthyl-6H-2-oxa-6-aza-cyclopenta[c]fluorène-1,3-dione

Le produit attendu est obtenu à partir du composé précédent selon le procédé décrit au stade G de la préparation A.

**PREPARATION Q : N,N'-bis[2-(1(R)-méthyl-aminoéthyl)éthane]1,2-diamine**

Le produit attendu est préparé à partir de N-(*tert*-butoxycarbonyl)-D-alanine et d'éthylènediamine selon la méthode décrite dans J. Med. Chem, 40, 449, 1997.

**EXEMPLE 1 : N,N'-bis[2-(2-aza-1,3-dioxo-10-méthoxy-cyclopenta[c]phénanthrène-2-yl)éthyl]éthane-1,2-diamine, bisméthanesulfonate**

A 80 mmoles du composé décrit dans la préparation A dans 1 l de toluène agitées à 80°C, sont ajoutées,

goutte à goutte, une solution de 40 mmoles N-1-[2-(2-aminoéthylamino)éthyl]éthane-1,2-diamine dans 20 ml de toluène. Le mélange réactionnel est ensuite porté au reflux 17 h, filtré chaud puis concentré sous vide. Le résidu, repris par 300 ml d'éthanol, est agité au reflux pendant 3 h, puis filtré chaud. Après retour à l'ambiante, le produit brut est isolé par filtration, puis purifié par chromatographie sur silice. Le produit sous forme base ainsi recueilli est agité dans 450 ml de dichlorométhane. On ajoute à ce milieu une solution de 2 équivalents d'acide méthansulfonique dans du dichlorométhane et on maintient sous agitation pendant 5 heures. Le produit est ensuite filtré, lavé à l'éther et séché.

**EXEMPLE 2 : N,N'-bis[2-(2-aza-1,3-dioxo-10-méthoxy-cyclopenta[c]phénanthrène-2-yl) éthyl]propane-1,3-diamine, bisméthanesulfonate**

Le produit attendu est obtenu selon le procédé décrit dans l'exemple 1 à partir du composé décrit dans la préparation A et de la N-1-[3-(2-aminoéthylamino)propyl]éthane-1,2-diamine.

**EXEMPLE 3 : 1,8-bis(2-Aza-1,3-dioxo-10-méthoxy-cyclopenta[c]phénanthrène-2-yl)-4-aza-octane, méthanesulfonate**

Le produit attendu est obtenu selon le procédé décrit dans l'exemple 1 à partir du composé décrit dans la préparation A et de la 4-aza-octane-1,8-diamine.
*Point de fusion : 294°C*

**EXEMPLE 4 : N,N-bis[3-(2-aza-1,3-dioxo-10-méthoxy-cyclopenta[c]phénanthrène-2-yl) propyl]méthylamine, méthanesulfonate**

Le produit attendu est obtenu selon le procédé décrit dans l'exemple 1 à partir du composé décrit dans la préparation a et de la N,N-bis(3-aminopropyl)méthylamine.

**EXEMPLE 5 : N,N'-bis[2-(2-aza-1,3-dioxo-cyclopenta[c]phénanthrène-2-yl)éthyl] propane-1,3-diamine, bisméthanesulfonate**

Le produit attendu est obtenu selon le procédé décrit dans l'exemple 1 à partir du composé décrit dans la préparation B et de la N-1-[3-(2-aminoéthylamino)propyl]éthane-1,2-diamine.

| *Microanalyse élémentaire :* | | | | |
|---|---|---|---|---|
| | *C %* | *H %* | *N %* | *S %* |
| *calculé* | *60,58* | *4,96* | *6,89* | *7,89* |
| *trouvé* | *60,79* | *4,91* | *6,83* | *7,79* |

**EXEMPLE 6 : N,N'-bis[2-(2-aza-1,3-dioxo-cyclopenta[c]phénanthrène-2-yl)éthyl] éthane-1,2-diamine, bisméthanesulfonate**

Le produit attendu est obtenu selon le procédé décrit dans l'exemple 1 en remplacant le produit décrit dans la préparation A par le produit décrit dans la préparation B.

| *Microanalyse élémentaire :* | | | | |
|---|---|---|---|---|
| | *C %* | *H %* | *N %* | *S %* |
| *calculé* | *60,14* | *4,79* | *7,01* | *8,03* |
| *trouvé* | *60,32* | *4,89* | *6,97* | *8,02* |

**EXEMPLE 7 : N,N'-bis[2-(2-aza-1,3-dioxo-naphto[2,3-c]furane-2-yl]éthyl]éthane-1,2-diamine, bisméthanesulfonate**

Le produit attendu est obtenu selon le procédé décrit dans l'exemple 1 en remplacant le produit décrit dans la préparation A par le produit décrit dans la préparation C.

| Microanalyse élémentaire : | | | | |
|---|---|---|---|---|
| | C % | H % | N % | S % |
| calculé | 55,01 | 4,90 | 8,02 | 9,18 |
| trouvé | 54,40 | 4,97 | 7,77 | 8,32 |

**EXEMPLE 8 : N,N'-bis[2-(2-aza-1,3-dioxo-8-thia-dicyclopenta[a,h]naphtalène-2-yl)éthyl]éthane-1,2-diamine, bisméthanesulfonate**

Le produit attendu est obtenu selon le procédé décrit dans l'exemple 1 en remplacant le produit décrit dans la préparation A par le produit décrit dans la préparation D.

| Microanalyse élémentaire : | | | | |
|---|---|---|---|---|
| | C % | H % | N % | S % |
| calculé | 45,50 | 4,22 | 5,59 | 19,18 |
| trouvé | 45,96 | 4,11 | 5,63 | 19,88 |

**EXEMPLE 9 : N,N-bis[3-(2-aza-1,3-dioxo-8-thia-dicyclopenta[a,h]naphtalène-2-yl)propyl]méthylamine, méthanesulfonate**

Le produit attendu est obtenu selon le procédé décrit dans l'exemple 1 à partir du composé décrit dans la préparation D et de la N,N-bis(3-aminopropyl)méthylamine.

**EXEMPLE 10 : N,N'-bis[2-(2,9-diaza-9,10-diméthyl-1,3-dioxo-6-méthoxy-9H-cyclopenta[b]fluorène-2-yl)éthyl] éthane-1,2-diamine, bisméthanesulfonate**

Le produit attendu est obtenu selon le procédé décrit dans l'exemple 1 en remplacant le produit décrit dans la préparation A par le produit décrit dans la préparation E et en opérant dans de l'éthanol à la place du toluène.

| Microanalyse élémentaire : | | | | |
|---|---|---|---|---|
| | C % | H % | N % | S % |
| calculé | 56,71 | 5,42 | 9,41 | 7,18 |
| trouvé | 56,71 | 5,61 | 9,51 | 7,11 |

**EXEMPLE 11 : N,N'-bis[2-(2,9-diaza-9,10-diméthyl-1,3-dioxo-6-méthoxy-9H-cyclopenta[b]fluorène-2-yl)éthyl] propane-1,3-diamine, bisméthanesulfonate**

Le produit attendu est obtenu selon le procédé décrit dans l'exemple 1 à partir du composé décrit dans la préparation E et de la N-1[3-(2-amino-éthylamino)propyl]éthane-1,2-diamine et en opérant dans de l'éthanol à la place du toluène.

| Microanalyse élémentaire : | | | | |
|---|---|---|---|---|
| | C % | H % | N % | S % |
| calculé | 56,94 | 5,56 | 9,27 | 7,02 |
| trouvé | 57,49 | 5,61 | 9,35 | 7,13 |

**EXEMPLE 12 : N,N-bis[3-(2,9-diaza-9,10-diméthyl-1,3-dioxo-6-méthoxy-9H-cyclopenta[b]fluorène-2-yl)propyl] méthylamine, méthanesulfonate**

Le produit attendu est obtenu selon le procédé décrit dans l'exemple 1 à partir du composé décrit dans la préparation E et de la N,N-bis(3-aminopropyl)méthylamine et en opérant dans de l'éthanol à la place du toluène.

| *Microanalyse élémentaire :* | | | | |
|---|---|---|---|---|
| | *C %* | *H %* | *N %* | *S %* |
| *calculé* | *63,38* | *5,70* | *8,80* | *4,03* |
| *trouvé* | *64,19* | *5,82* | *9,18* | *4,03* |

**EXEMPLE 13 : 1,8-bis(2,9-diaza-9,10-diméthyl-1,3-dioxo-6-méthoxy-9H-cyclopenta[b]fluorène-2-yl)4-azaoctane, méthanesulfonate**

Le produit attendu est obtenu selon le procédé décrit dans l'exemple 1 à partir du composé décrit dans la préparation E et de la 4-aza-octane-1,8-diamine et en opérant dans de l'éthanol à la place du toluène.

| *Microanalyse élémentaire :* | | | | |
|---|---|---|---|---|
| | *C %* | *H %* | *N %* | *S %* |
| *calculé* | *63,38* | *5,70* | *8,80* | *4,03* |
| *trouvé* | *62,95* | *5,80* | *8,96* | *4,02* |

**EXEMPLE 14 : N,N-bis[3-(2,9-diaza-9,10-diméthyl-1,3-dioxo-6-méthoxy-9H-cyclopenta[b]fluorène-2-yl)propyl] amine, méthanesulfonate**

Le produit attendu est obtenu selon le procédé décrit dans l'exemple 1 à partir du composé décrit dans la préparation E et de la N,N-bis(3-aminopropyl)amine et en opérant dans de l'éthanol à la place du toluène.

| *Microanalyse élémentaire :* | | | | |
|---|---|---|---|---|
| | *C %* | *H %* | *N %* | *S %* |
| *calculé* | *62,98* | *5,54* | *8,96* | *4,10* |
| *trouvé* | *62,91* | *5,63* | *8,78* | *4,17* |

**EXEMPLE 15 : N,N'-bis[3-(2,9-diaza-9,10-diméthyl-1,3-dioxo-6-méthoxy-9H-cyclopenta[b]fluorène-2-yl)propyl] éthane-1,2-diamine, bisméthanesulfonate**

Le produit attendu est obtenu selon le procédé décrit dans l'exemple 1 à partir du produit décrit dans la préparation E et de la N-[2-(3-aminopropylamino)éthyl]propane-1,3-diamine en opérant dans de l'éthanol à la place du toluène.

| *Microanalyse élémentaire :* | | | | |
|---|---|---|---|---|
| | *C %* | *H %* | *N %* | *S %* |
| *calculé* | *57,38* | *5,69* | *9,12* | *6,96* |
| *trouvé* | *57,08* | *5,66* | *8,64* | *6,50* |

**EXEMPLE 16 : N,N'-bis[2-(2-aza-1,3-dioxo-10-hydroxy-cyclopenta[c]phénanthrène-2-yl)éthyl]éthane-1,2-diamine, bromhydrate**

Une suspension de 1 mmole du composé de l'exemple 1 dans 40 ml d'acide bromhydrique à 47 % est portée au reflux 8 heures. Après retour à l'ambiante, le solide est filtré, lavé à l'eau et repris dans de l'éthanol à reflux pendant 1 h. Après filtration, on obtient le produit attendu.

**EXEMPLE 17 : N,N-bis[3-(2-aza-1,3-dioxo-10-hydroxy-cyclopenta[c]phénanthrène-2-yl) propyl]méthylamine, bromhydrate**

Le produit attendu est obtenu selon le procédé décrit dans l'exemple 16 à partir du composé de l'exemple 4.

**EXEMPLE 18 : N,N'-bis[2-(2,9-diaza-9,10-diméthyl-1,3-dioxo-6-hydroxy-9H-cyclopenta[b]fluorène-2-yl)éthyl] éthane-1,2-diamine, dibromhydrate**

Le produit attendu est obtenu selon le procédé décrit dans l'exemple 16 à partir du composé de l'exemple 10.

**EXEMPLE 19 : N,N'-bis[2-(2,9-diaza-9,10-diméthyl-1,3-dioxo-6-hydroxy-9H-cyclopenta[b]fluorène-2-yl)éthyl] propane-1,3-diamine, dibromhydrate**

Le produit attendu est obtenu selon le procédé décrit dans l'exemple 16 à partir du composé de l'exemple 11.

| *Microanalyse élémentaire :* | | | | |
|---|---|---|---|---|
| | *C %* | *H %* | *N %* | *Br %* |
| *calculé* | *55,20* | *4,75* | *9,90* | *18,83* |
| *trouvé* | *55,11* | *4,44* | *9,73* | *17,53* |

**EXEMPLE 20 : 1,8-bis(2,9-diaza-9,10-diméthyl-1,3-dioxo-6-hydroxy-9H-cyclopenta[b]fluorène-2-yl)4-azaoctane, bromhydrate**

Le produit attendu est obtenu selon le procédé décrit dans l'exemple 16 à partir du composé de l'exemple 13.

| *Microanalyse élémentaire* : | | | | |
|---|---|---|---|---|
| | *C %* | *H %* | *N %* | *Br %* |
| *calculé* | *62,24* | *5,09* | *9,30* | *10,62* |
| *trouvé* | *63,04* | *5,02* | *9,25* | *9,57* |

**EXEMPLE 21 : N,N'-bis[3-(2,9-diaza-9,10-diméthyl-1,3-dioxo-6-hydroxy-9H-cyclopenta[b]fluorène-2-yl)propyl] éthane-1,2-diamine**

Le produit attendu est obtenu selon le procédé décrit dans l'exemple 16 à partir du composé de l'exemple 15.

| *Microanalyse élémentaire :* | | | | |
|---|---|---|---|---|
| | *C%* | *H%* | *N%* | *Br%* |
| *calculé* | *55,70* | *4,91* | *9,74* | *18,53* |
| *trouvé* | *55,88* | *4,68* | *9,13* | *17,87* |

**EXEMPLE 22 : 1-[2-(1,3-dioxo-2,3-dihydro-5-nitro-benzo[d,e]isoquinolin-2-yl)éthyl]-3-[2-(2-aza-1,3-dioxo-10-méthoxy-cyclopenta[c]phénanthrène-2-yl)-éthyl] propane-1,3-diamine, bisméthanesulfonate**

Stade A : 2-[2-(3-aminopropylamino)éthylamino]-5-nitro-benzo[d,e]isoquinoline-1,3-dione, triméthanesulfonate

A une solution agitée à 0°C de 50 mmoles de N,N'-bis(2-aminoéthyl)propane-1,3-diamine dans 200 ml de THF, on ajoute goutte à goutte une solution à 0°C de 50 mmoles d'anhydride 3-nitro-naphtalique dans 300 ml de THF. L'addition terminée, le mélange réactionnel est agité 3 heures à température ambiante, 2 heures au reflux puis est filtré chaud. Le filtrat est agité à l'ambiante et on ajoute goutte à goutte 150 mmoles d'acide méthanesulfonique dans 100 ml de THF. Le précipité est filtré, repris dans 1 l d'éthanol et porté au reflux 2 heures. L'insoluble est filtré et le filtrat est refroidi. Le produit désiré est obtenu après filtration avec un rendement de 32 %.

Stade B : 1-[2-(1,3-dioxo-2,3-dihydro-5-nitro-benzo[d,e]isoquinolin-2-yl)éthyl]-3-[2-(2-aza-1,3-dioxo-10-méthoxy-cyclopenta[c]phénanthrène-2-yl)-éthyl] propane-1,3-diamine, bisméthanesulfonate

A une solution de 5 mmoles du composé préparé au stade précédent et de 15 mmoles de diisopropyléthylamine dans 1 l d'éthanol agitée à température ambiante, on ajoute par petites fractions 5 mmoles du composé décrit dans la préparation A. L'addition terminée, on porte au reflux 15 heures, puis on filtre à chaud. Le filtrat refroidi est filtré et le solide est purifié par chromatographie sur silice éluant $CH_2Cl_2$95 / MeOH5 / $NH_2OH$0,1. Les fractions con-

tenant le produit sont rassemblées, concentrées, le résidu est repris dans 1 l de chlorure de méthylène et on ajoute 2 équivalents d'acide méthanesulfonique. Le produit est isolé par filtration.

**EXEMPLE 23 : 1-[2-(1,3-dioxo-2,3-dihydro-5-nitro-benzo[d,e]isoquinolin-2-yl)éthyl]-2-[2-(2-aza-1,3-dioxo-10-méthoxy-cyclopenta[c]phénanthrène-2-yl) éthyl]éthane-1,2-diamine, bisméthanesulfonate**

Le produit attendu est obtenu selon le procédé décrit dans l'exemple 22 en utilisant au stade A la N,N'-bis (2-aminoéthyl)éthane-1,2-diamine.

| *Microanalyse élémentaire :* | | | | |
|---|---|---|---|---|
| | *C %* | *H %* | *N %* | *S %* |
| *calculé* | *53,00* | *4,52* | *8,31* | *8,37* |
| *trouvé* | *53,00* | *4,64* | *8,40* | *8,54* |

**EXEMPLE 24 : N,N'-bis[2-(2-aza-6-oxa-cyclopenta[c]fluorène-1,3-dione-2-yl)éthyl] propane-1,3-diamine, bisméthanesulfonate**

Le produit attendu est obtenu selon le procédé décrit dans l'exemple 1 à partir du composé décrit dans la préparation I et de la N-1-[3-(2-aminoéthylamino)propyl]-éthane-1,2-diamine.

| *Microanalyse élémentaire :* | | | | |
|---|---|---|---|---|
| | *C%* | *H%* | *N%* | *S%* |
| *calculé* | *56,05* | *4,58* | *7,07* | *8,09* |
| *trouvé* | *56,15* | *4,48* | *7,02* | *8,30* |

**EXEMPLE 25 : N,N'-bis[2-(2-aza-1,3-dioxo-10-nitro-cyclopenta[c]phénanthrène-2-yl) éthyl]propane-1,3-diamine, bisméthanesulfonate**

Le produit attendu est obtenu selon le procédé décrit dans l'exemple 1 à partir du composé décrit dans la préparation F et de la N-1-[3-(2-aminoéthylamino)propyl]-éthane-1,2-diamine.

| *Microanalyse élémentaire :* | | | | |
|---|---|---|---|---|
| | *C%* | *H%* | *N%* | *S%* |
| *calculé* | *54,54* | *4,24* | *9,31* | *7,10* |
| *trouvé* | *54,73* | *4,27* | *9,24* | *7,24* |

**EXEMPLE 26 : N,N'-bis[2-(2-aza-10-oxa-1,3-dioxo-cyclopenta[a]fluorène-2-yl)éthyl]propane-1,3-diamine, bisméthanesulfonate**

Le produit attendu est obtenu selon le procédé décrit dans l'exemple 1 à partir du composé décrit dans la préparation H et de la N-1-[3-(2-aminoéthylamino)propyl]-éthane-1,2-diamine.

| *Microanalyse élémentaire :* | | | | |
|---|---|---|---|---|
| | *C%* | *H%* | *N%* | *S%* |
| *calculé* | *56,05* | *4,58* | *7,07* | *8,09* |
| *trouvé* | *56,48* | *4,66* | *7,03* | *7,85* |

**EXEMPLE 27 : N,N'-bis[2-(2-aza-1,3-dioxo-10-méthoxy-cyclopenta[c]phénanthrène-2-yl)(1(R)-méthyl)-éthyl] éthane-1,2-diamine, bisméthanesulfonate**

Le produit attendu est obtenu selon le procédé décrit dans l'exemple 1 à partir du composé décrit dans la préparation A et du composé décrit dans la préparation Q.

| Microanalyse élémentaire : | | | | |
|---|---|---|---|---|
| | C% | H% | N% | S% |
| calculé<br>trouvé | 59,58<br>59,77 | 5,23<br>5,26 | 6,32<br>6,15 | 7,23<br>6,99 |

**EXEMPLE 28 : N,N'-bis[2-(2-aza-1,3-dioxo-cyclopenta[b]phénanthrène-2-yl)éthyl]propane-1,3-diamine bisméthanesulfonate**

Le produit attendu est obtenu selon le procédé décrit dans l'exemple 1 à partir du composé décrit dans la préparation G et de la N-1-[3-(2-aminoéthylamino)propyl]-éthane-1,2-diamine.

**EXEMPLE 29 : N,N'-bis[2-(2-aza-1,3-dioxo-10-méthyl-10*H*-pyrrolo[3,4-a]carbazole-2-yl)éthyl]propane-1,3-diamine, bisméthanesulfonate**

Le produit attendu est obtenu selon le procédé décrit dans l'exemple 1 à partir du composé décrit dans la préparation K et de la N-1-[3-(2-aminoéthylamino)propyl]-éthane-1,2-diamine.

| Microanalyse élémentaire : | | | | |
|---|---|---|---|---|
| | C% | H% | N% | S% |
| calculé<br>trouvé | 57,20<br>57,18 | 5,17<br>5,09 | 10,26<br>9,92 | 7,83<br>8,04 |

**EXEMPLE 30 : N,N'-bis[2-(2-aza-1,3-dioxo-6-thia-cyclopenta[c]fluorène-2-yl)éthyl] propane-1,3-diamine, bisméthanesulfonate**

Le produit attendu est obtenu selon le procédé décrit dans l'exemple 1 à partir du composé décrit dans la préparation J et de la N-1-[3-(2-aminoéthylamino)propyl]-éthane-1,2-diamine.

| Microanalyse élémentaire : | | | | |
|---|---|---|---|---|
| | C% | H% | N% | S% |
| calculé<br>trouvé | 53,87<br>53,26 | 4,40<br>4,81 | 6,79<br>6,88 | 15,55<br>15,88 |

**EXEMPLE 31 : N,N'-bis[2-(2-aza-1,3-dioxo-6-méthyl-6*H*-pyrrolo[3,4-c]carbazole-2-yl) éthyl]propane-1,3-diamine, bisméthanesulfonate**

Le produit attendu est obtenu selon le procédé décrit dans l'exemple 1 à partir du composé décrit dans la préparation L et de la N-1-[3-(2-aminoéthylamino)propyl]-éthane-1,2-diamine.

| Microanalyse élémentaire : | | | | |
|---|---|---|---|---|
| | C% | H% | N% | S% |
| calculé<br>trouvé | 57,20<br>57,18 | 5,17<br>5,09 | 10,26<br>9,92 | 7,83<br>8,04 |

**EXEMPLE 32 : N,N'-bis[2-(2-aza-1,3-dioxo-10-méthoxy-cyclopenta[c]phénanthrène-2yl)éthyl]butane-1,4-diamine, bisméthanesulfonate**

Le produit attendu est obtenu selon le procédé décrit dans l'exemple 1 à partir du composé décrit dans la préparation A et de la N-1-[4-(2-aminoéthylamino)butyl]éthane-1,2-diamine.

| Microanalyse élémentaire : | | | | |
|---|---|---|---|---|
| | C% | H% | N% | S% |
| calculé | 59,58 | 5,23 | 6,32 | 7,23 |
| trouvé | 59,17 | 5,37 | 6,26 | 6,98 |

**EXEMPLE 33 : N,N'-bis[2-(2-aza-1,3-dioxo-6*H*-pyrrolo[3,4-c]carbazole-2-yl)éthyl] propane-1,3-diamine, bisméthanesulfonate**

Le produit attendu est obtenu selon le procédé décrit dans l'exemple 1 à partir du composé décrit dans la préparation M et de la N-1-[3-(2-aminoéthylamino)propyl]-éthane-1,2-diamine.

| Microanalyse élémentaire : | | | | |
|---|---|---|---|---|
| | C% | H% | N% | S% |
| calculé | 56,19 | 4,84 | 10,63 | 8,11 |
| trouvé | 56,34 | 5,08 | 10,71 | 8,24 |

**EXEMPLE 34 : 1-[2-(1,3-dioxo-2,3-dihydro-5-nitro-benzo[d,e]isoquinolin-2-yl)éthyl]-3-[2-(2-aza-1,3-dioxo-6-méthyl-6*H*-pyrrolo[3,4-c]carbazole-2-yl)éthyl] propane-1,3-diamine, bisméthanesulfonate**

Le produit attendu est obtenu selon le procédé décrit dans l'exemple 22 en utilisant au stade B le composé décrit dans la préparation L .

| Microanalyse élémentaire : | | | | |
|---|---|---|---|---|
| | C% | H% | N% | S% |
| calculé | 53,33 | 4,72 | 10,36 | 7,91 |
| trouvé | 53,80 | 4,70 | 10,46 | 7,88 |

**EXEMPLE 35 : N,N'-bis[2-(2-aza-1,3-dioxo-6-méthyl-9-méthoxy-*6H*-pyrrolo[3,4-c] carbazole)-2-yl]propane-1,3-diamine, bisméthanesulfonate**

Le produit attendu est obtenu selon le procédé décrit dans l'exemple 1 en utilisant le composé décrit dans la préparation O et la N-1-[3-(2-aminoéthylamino)propyl]éthane-1,2-diamine.

**EXEMPLE 36 : N,N'-bis[2-(2-aza-1,3-dioxo-6-méthyl-*6H*-pyrrolo[3,4-c]carbazole-2-yl) (1(R)-méthyl)éthyl]éthane-1,2-diamine, bisméthanesulfonate**

Le produit attendu est obtenu selon le procédé décrit dans l'exemple 1 en utilisant le composé décrit dans la préparation L et celui décrit dans la préparation Q.

**EXEMPLE 37 : 1-[2-(1,3-Dioxo-2,3-dihydro-5-nitro-benzo[d,e]isoquinoline-2-yl) (1(R)-méthyl)éthyl]-2-[2-(2-aza-1,3-dioxo-6-méthyl-*6H*-pyrrolo[3,4-c] carbazole-2-yl)(1(R)-méthyl)éthyl]éthane-1,2-diamine, bisméthanesulfonate**

Le produit attendu est obtenu selon le procédé décrit dans l'exemple 22 en utilisant au stade B le composé décrit dans la préparation L.

**EXEMPLE 38 : N,N'-bis[2-(2-aza-1,3-dioxo-6-méthyl-*6H*-9-nitro-pyrrolo[3,4-c] carbazole-2-yl)(1(R)-méthyl) éthyl]éthane-1,2-diamine, bisméthanesulfonate**

Le produit attendu est obtenu selon le procédé décrit dans l'exemple 1 en utilisant le composé décrit dans la préparation N.

**EXEMPLE 39 : N,N'-bis[2-(2-aza-5,6-diméthyl-1,3-dioxo-*6H*-pyrrolo[3,4,c]carbozole-2-yl)(1(R)-méthyl)éthyl] éthane-1,2-diamine, bisméthanesulfonate**

Le produit attendu est obtenu selon le procédé décrit dans l'exemple 1 en utilisant le composé décrit dans la préparation P.

**EXEMPLE 40 : 1-[2-(2-aza-1,3-dioxo-6-méthyl]-6H-pyrrolo[3,4-c]carbazole-2-yl)(1(R)-méthyl)éthyl]-2-[2-(2-aza-1,3-dioxo-10-méthoxycyclopenta[c] phénanthrène-2-yl)(1(R)-méthyl)éthyl]éthane-1,2-diamine, bisméthanesulfonate**

Le produit attendu est obtenu selon le procédé décrit dans l'exemple 22 en utilisant au stade A le composé décrit dans la préparation A et au stade B le composé décrit dans la préparation L.

**ETUDE PHARMACOLOGIQUE DES DERIVES DE L'INVENTION**

**EXEMPLE 41 : Prolifération in vitro**

Ce test permet de mesurer le pouvoir antiprolifératif d'un composé in vitro en déterminant la concentration de produit inhibant 50 % la croissance cellulaire comparativement aux cellules témoins non traitées ($IC_{50}$).
Il s'agit d'un test colorimétrique reposant sur le clivage, par réduction, par la succinate déshydrogénase mitochondriale des cellules vivantes d'un sel soluble de tétrazolium le 3-(4,5-diméthylthiazol-2-yl) 2,5-diphényltétrazolium bromide, ou MTT, en cristaux de formazan, pourpres et insolubles dans le milieu de culture.
Afin d'assurer leur parfaite adhérence, les cellules sont ensemencées à la densité de 625 cellules par puits dans des plaques 96 puits, 24 heures avant d'être incubées à 37°C, en continu, et en présence d'une gamme de concentrations du produit à tester. Après 4 temps de doublement de la population cellulaire (détermination après étude de la cinétique de croissance de lignée), les cellules vivantes sont colorées par le MTT (1/10ème du volume de milieu est ajouté dans le puits, solution à 5 mg/ml dans du PBS) pendant 4 heures à 37°C. Au terme de cette incubation, on rajoute un volume égal à celui du puits d'une solution de SDS (SDS 20 %, diméthyl formamide 50 % dans l'eau, pH 4,7) qui après une exposition d'une nuit solubilise les cristaux de formazan. La densité optique du milieu est alors mesurée à 540 nm par un lecteur de plaques Titertek multiscan MCC (Labsystem).
Des droites de calibration ont été réalisées pour chacune des lignées à partir de cellules ensemencées à densités croissantes et ont permis d'établir une relation linéaire entre le nombre de cellules vivantes et la densité optique lue.
Les lignées utilisées dans ce test sont :

- la lignée LLC : carcinome pulmonaire de Lewis d'origine murin,
- la lignée HT-29 : carcinome de colon d'origine humaine.

Les résultats obtenus sur ces deux lignées montrent que les composés de l'invention possèdent un très fort pouvoir antiprolifératif.
A titre d'exemple, l'$IC_{50}$ du composé de l'exemple 22 est égale à 53,60 nM sur la lignée LLC et à 1,40 nM sur la lignée HT-29.

**EXEMPLE 42 : Activité antitumorale in vivo**

L'activité antitumorale des composés de l'invention a été étudiée chez la souris Nude. Des fragments du carcinome épidermoide humain KB-3-1 ont été greffés par voie sous-cutanée sur des souris Nude Swiss femellles agées de 4 à 6 semaines et pesant de 20 à 22 g. Lorsque les tumeurs atteignent un diamètre de 6 mm environ, les souris sont distribuées dans les groupes contrôle et traité (7 souris par groupe) de façon à obtenir un volume tumoral médian identique dans chacun des groupes. Le produit à tester est administré par voie i.v., 1 injection par jour pendant 5 jours. Les tumeurs sont mesurées 2 fois par semaine, le volume tumoral est calculé selon la formule : volume = (longueur x largeur$^2$)/2. L'activité antitumorale est évaluée grâce à 2 paramètres : le T/C médian, exprimé en pourcentage et le SGD (Specific growth delay).
T/C médian (%) à un temps t = 100 x (Vt/V0) médian traité/ (Vt/V0) médian contrôle avec Tt : volume tumoral au temps t ; V0 : volume tumoral au début du traitement.
SGD = (Td traité - Td contrôle) / Td contrôle avec Td = temps de doublement médian de la tumeur.
Les résultats obtenus avec les produits de l'invention montrent qu'ils inhibent fortement la croissance tumorale.
A titre d'exemple, le composé de l'exemple 1, administré à la dose de 15 mg/kg par voie i.v. pendant 5 jours, donne les résultats suivants : 14 jours après le début du traitement, le T/C médian est de 26 % (donc 74 % d'inhibition) et le

SGD est de 1.3. De plus, le traitement n'induit pas de perte de poids, il est donc bien toléré.

**EXEMPLE 43 : Composition pharmaceutique**

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

**1.** Composés de formule (I) :

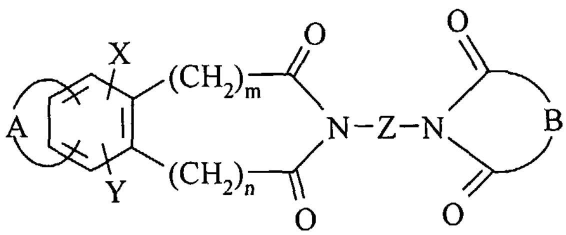

(I)

dans laquelle :

m, n, identiques ou différents, représentent 0 ou 1,

X, Y, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

Z représente une chaîne alkylène $C_4$ à $C_{12}$ linéaire ou ramifié dans laquelle un ou plusieurs groupements $-CH_2-$ sont éventuellement remplacés par l'un quelconque des atomes ou groupements : -NR- (dans lequel R représente un atome d'hydrogène ou un groupement alkyle ($C_1$-$C_6$) linéaire ou ramifié), -O-, -S-, -SO-, $-SO_2-$, -CONH-, ou par un groupement hétérocyclique substitué ou non,

A forme avec deux atomes de carbone adjacents du cycle phényle :

- un cycle phényle substitué ou non,
- un cycle naphtyle substitué ou non,
- un cycle tétrahydronaphtyle substitué ou non, ou 1,4-dioxo-1,2,3,4-tétrahydronapthyle substitué ou non,
- ou, un hétérocycle substitué ou non,

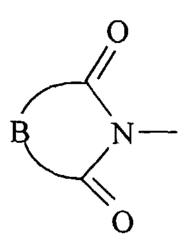

représente l'un quelconque des groupements suivants :

*

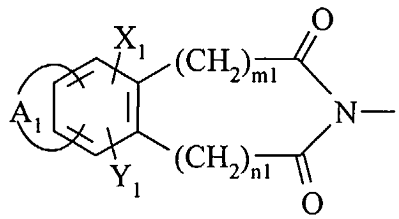

dans lequel :

$m_1$, $n_1$, identiques ou différents, représentent 0 ou 1,

$X_1$, $Y_1$, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

$A_1$ forme avec deux atomes de carbone adjacents du cycle phényle :

- un cycle phényle substitué ou non,
- un cycle naphtyle substitué ou non,
- un cycle tétrahydronaphtyle substitué ou non, ou 1,4-dioxo-1,2,3,4-tétrahydronapthyle substitué ou non,
- ou, un hétérocycle substitué ou non,

*

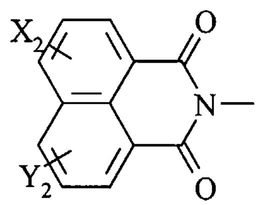

dans lequel $X_2$, $Y_2$, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

leurs stéréoisomères ainsi que leurs sels d'addition à un acide ou à une base pharmaceutiquement acceptable, étant entendu que :

- par groupement phényle substitué ou non, naphtyle substitué ou non, tétrahydronaphtyle substitué ou non, on entend groupement phényle, napthyle ou tétrahydronaphtyle éventuellement substitué par un ou plusieurs atomes d'halogène ou groupements alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, nitro, cyano ou amino (substitué éventuellement par un ou plusieurs groupements alkyle ($C_1$-$C_6$) linéaire ou ramifié),

- par hétérocycle substitué ou non, on entend un groupement mono ou bicyclique, saturé ou insaturé, de 5 à 16 chaînons contenant un, deux ou trois hétéroatomes choisis parmi oxygène, azote ou soufre, étant entendu que l'hétérocycle peut être éventuellement substitué par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle, nitro, cyano ou amino (substitué éventuellement par un ou plusieurs groupements alkyle ($C_1$-$C_6$) linéaire ou ramifié).

**2.** Composés de formule (I) selon la revendication 1 tels que A représente avec deux atomes de carbone adjacents du cycle phényle, un cycle naphtyle substitué ou non.

**3.** Composés de formule (I) selon la revendication 1 tels que A représente avec deux atomes de carbone adjacents du cycle phényle, un hétérocycle substitué ou non choisi parmi les cycles indole éventuellement substitué, benzo [b]thiophène éventuellement substitué ou benzo[b]furane éventuellement substitué.

**4.** Composés de formule (I) selon la revendication 1 tels que Z représente une chaîne alkylène $C_4$ à $C_{12}$ dans laquelle 1, 2 ou 3 groupements $-CH_2-$ sont remplacés par 1, 2 ou 3 groupements -NR- (dans lequel R représente un atome d'hydrogène ou un groupement alkyle ($C_1$-$C_6$) linéaire ou ramifié).

**5.** Composés de formule (I) selon la revendication 1 tels que

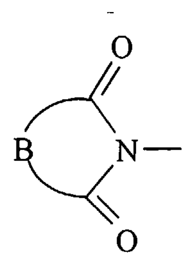

représente le groupement

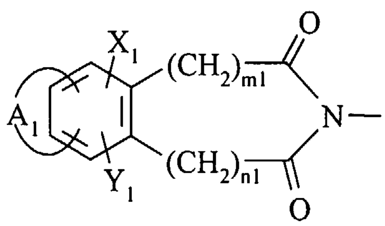

dans lequel :

$m_1$, $n_1$, identiques ou différents, représentent 0 ou 1,

$X_1$, $Y_1$, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,

$A_1$ forme avec deux atomes de carbone adjacents du cycle phényle :

- un cycle phényle substitué ou non,
- un cycle naphtyle substitué ou non,
- un cycle tétrahydronaphtyle substitué ou non, ou 1,4-dioxo-1,2,3,4-tétrahydronapthyle substitué ou non,
- ou, un hétérocycle substitué ou non,

leurs stéréoisomères ainsi que leurs sels d'addition à un acide ou à une base pharmaceutiquement acceptable.

**6.** Composés de formule (I) selon la revendication 1 tels que

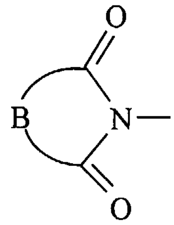

représente le groupement

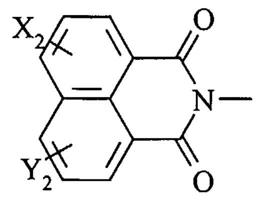

dans lequel $X_2$, $Y_2$, identiques ou différents, représentent un atome d'hydrogène, d'halogène, un groupement alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, hydroxy, cyano, nitro, amino, alkylamino ou dialkylamino,
leurs stéréoisomères ainsi que leurs sels d'addition à un acide ou à une base pharmaceutiquement acceptable.

7. Composé de formule (I) selon la revendication 1 tels que m et n représentent simultanément 0.

8. Composé de formule (I) selon la revendication 1 qui est le N,N'-bis[2-(2-aza-1,3-dioxo-10-méthoxy-cyclopenta[c] phénanthrène-2-yl)éthyl]éthane-1,2-diamine ainsi que ses sels d'addition à un acide pharmaceutiquement acceptable.

9. Composé de formule (I) selon la revendication 1 qui est le N,N'-bis[2-(2-aza-1,3-dioxo-10-méthoxy-cyclopenta[c] phénanthrène-2-yl)éthyl]propane-1,3-diamine ainsi que ses sels d'addition à un acide pharmaceutiquement acceptable.

10. Composé de formule (I) selon la revendication 1 qui est le 1-[2-(1,3-dioxo-2,3-dihydro-5-nitro-benzo[d,e]isoquinolin-2-yl)éthyl]-3-[2-(2-aza-1,3-dioxo-10-méthoxy-cyclopenta[c] phénanthrène-2-yl)-éthyl]propane-1,3-diamine ainsi que ses sels d'addition à un acide pharmaceutiquement acceptable.

11. Composé de formule (I) selon la revendication 1 qui est le 1-[2-(1,3-dioxo-2,3-dihydro-5-nitro-benzo[d,e]isoquinolin-2-yl)éthyl]-2-[2-(2-aza-1,3-dioxo-10-méthoxy-cyclopenta[c] phénanthrène-2-yl)éthyl]éthane-1,2-diamine ainsi que ses sels d'addition à un acide pharmaceutiquement acceptable.

12. Procédé de préparation des composés de formule (I) selon la revendication 1, caractérisé en ce que l'on utilise comme produit de départ un anhydride de formule (II) :

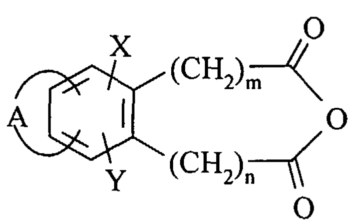

(II)

dans laquelle A, X, Y, m et n sont tels que définis dans la formule (I),
que l'on met en réaction avec un excès d'une diamine de formule (III) :

$$H_2N - Z - NH_2 \qquad (III)$$

dans laquelle Z est tel que défini dans la formule (I), pour conduire après séparation :

- soit au composé de formule (I/a), cas particulier des composés de formule (I) :

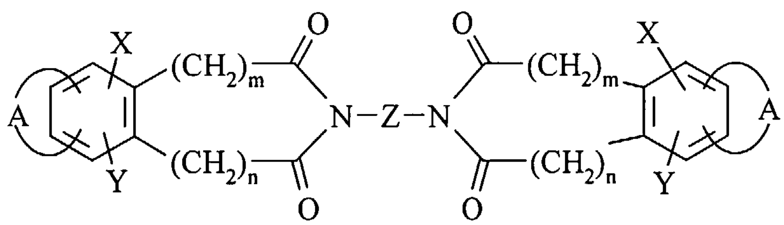

(I/a)

dans laquelle A, X, Y, Z, m et n ont la même signification que dans la formule (I),

- soit au composé de formule (IV) :

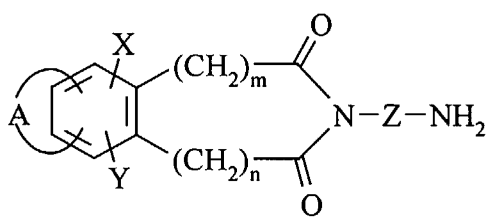

(IV)

dans laquelle A, X, Y, Z, m et n ont la même signification que dans la formule (I),

- que l'on fait réagir :

  * *soit* avec l'anhydride de formule (II/a) :

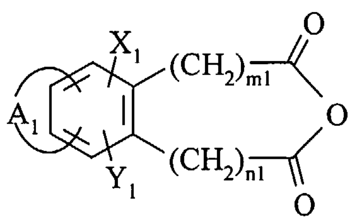

(II/a)

dans laquelle $A_1$, $X_1$, $Y_1$, $m_1$ et $n_1$ sont tels que définis dans la formule (I), pour conduire au composé de formule (I/b), cas particulier des composés de formule (I) :

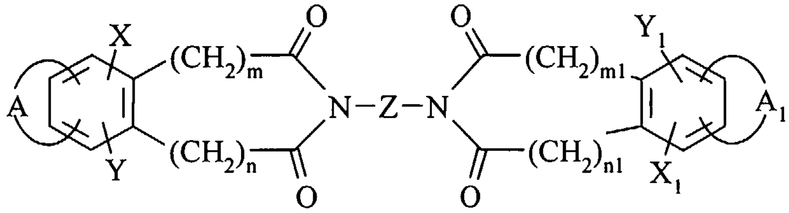

(I/b)

dans laquelle A, X, Y, m, n Z, $A_1$, $X_1$, $Y_1$, $m_1$, $n_1$ ont la même signification que dans la formule (I),

  * *soit* avec l'anhydride de formule (V) :

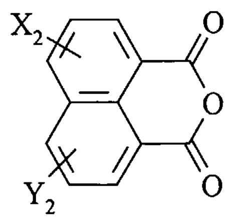

(V)

dans laquelle $X_2$ et $Y_2$ sont tels que définis dans la formule (I), pour conduire au composé de formule (I/c), cas particulier des composés de formule (I) :

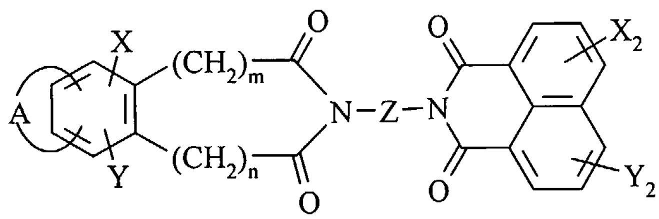

(I/c)

dans laquelle A, X, Y, $X_2$, $Y_2$, Z, m et n ont la même signification que dans la formule (I),

composé de formule (I/a), (I/b) ou (I/c), qui peut subir, si on le souhaite, les réactions classiques de transformation des substituants sur les noyaux aromatiques, que l'on purifie, le cas échéant, selon une technique classique de purification, dont on sépare éventuellement les stéréoisomères selon une technique classique de séparation et que l'on transforme, si on le souhaite, en ses sels d'addition à un acide ou à une base pharmaceutiquement acceptable.

**13.** Compositions pharmaceutiques contenant comme principe actif au moins un composé selon l'une quelconque des revendications 1 à 11, seul ou en combinaison avec un ou plusieurs excipients ou véhicules inertes, non toxiques, pharmaceutiquement acceptables.

**14.** Compositions pharmaceutiques selon la revendication 13 contenant au moins un principe actif selon l'une quelconque des revendications 1 à 11 utiles comme agents anticancéreux.

**Patentansprüche**

**1.** Verbindungen der Formel (I):

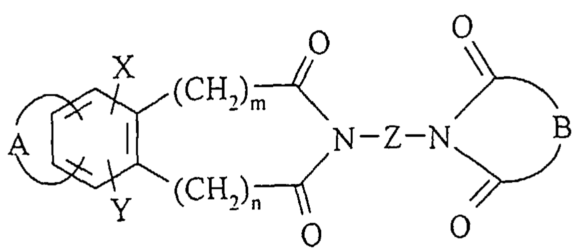

(I)

in der:

m und n, die gleichartig oder verschieden sind, 0 oder 1 bedeuten,

X und Y, die gleichartig oder verschieden sind, ein Wasserstoffatom, ein Halogenatom, eine geradkettige oder verzweigte ($C_1$-$C_6$)-Alkylgruppe, eine geradkettige oder verzweigte ($C_1$-$C_6$)-Trihalogenoalkylgruppe, eine geradkettige oder verzweigte ($C_1$-$C_6$)-Alkoxygruppe, eine Hydroxygruppe, eine Cyanogruppe, eine Nitrogruppe, eine Aminogruppe, eine Alkylaminogruppe oder eine Dialkylaminogruppe bedeuten,

Z eine geradkettige oder verzweigte $C_4$-$C_{12}$-Alkylenkette, in der eine oder mehrere Gruppen -$CH_2$- gegebenenfalls durch eines der Atome oder Gruppen: -NR- (worin R ein Wasserstoffatom oder eine geradkettige oder verzweigte ($C_1$-$C_6$)-Alkylgruppe darstellt), -O-, -S-, -SO-, -$SO_2$-, -CONH- oder durch eine gegebenenfalls substituierte heterocyclische Gruppe ersetzt sind, darstellt,

A mit den beiden benachbarten Kohlenstoffatomen des Phenylrings:

- einen gegebenenfalls substituierten Phenylring,
- einen gegebenenfalls substituierten Naphthylring,
- einen gegebenenfalls substituierten Tetrahydronaphthylring oder einen gegebenenfalls substituierten 1,4-Dioxo-1,2,3,4,-tetrahydronaphthylring,
- oder einen gegebenenfalls substituierten Heterocyclus bildet,

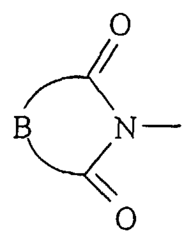

eine der folgenden Gruppen:

*

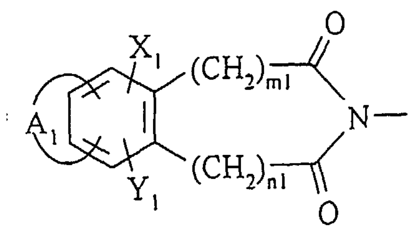

in der:

$m_1$ und $n_1$, die gleichartig oder verschieden sind, 0 oder 1 darstellen,

$X_1$ und $Y_1$, die gleichartig oder verschieden sind, ein Wasserstoffatom, ein Halogenatom, eine geradkettige oder verzweigte ($C_1$-$C_6$)-Alkylgruppe, eine geradkettige oder verzweigte ($C_1$-$C_6$)-Trihalogenoalkylgruppe, eine geradkettige oder verzweigte ($C_1$-$C_6$)-Alkoxygruppe, eine Hydroxygruppe, eine Cyanogruppe, eine Nitrogruppe, eine Aminogruppe, eine Alkylaminogruppe oder eine Dialkylaminogruppe darstellen,

$A_1$ mit den beiden benachbarten Kohlenstoffatomen des Phenylrings:

- einen gegebenenfalls substituierten Phenylring,
- einen gegebenenfalls substituierten Naphthylring,
- einen gegebenenfalls substituierten Tetrahydronaphthylring oder einen gegebenenfalls substituierten 1,4-Dioxo-1,2,3,4-tetrahydronaphthylring oder
- einen gegebenenfalls substituierten Heterocyclus bildet,

*

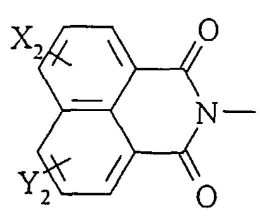

in der $X_2$ und $Y_2$, die gleichartig oder verschieden sind, ein Wasserstoffatom, ein Halogenatom, eine geradkettige oder verzweigte ($C_1$-$C_6$)-Alkylgruppe, eine geradkettige oder verzweigte ($C_1$-$C_6$)-

Trihalogenoalkylgruppe, eine geradkettige oder verzweigte ($C_1$-$C_6$)-Alkoxygruppe, eine Hydroxygruppe, eine Cyanogruppe, eine Nitrogruppe, eine Aminogruppe, eine Alkylaminogruppe oder eine Dialkylaminogruppe darstellen, bedeutet,

deren Stereoisomere sowie deren Additionssalze mit einer pharmazeutisch annehmbaren Base, wobei es sich versteht, daß:

- unter einer gegebenenfalls substituierten Phenylgruppe, gegebenenfalls substituierten Naphthylgruppe oder gegebenenfalls substituierten Tetrahydronaphthylgruppe eine Phenylgruppe, Naphthylgruppe oder Tetrahydronaphthylgruppe zu verstehen ist, die gegebenenfalls durch ein oder mehrere Halogenatome, geradkettige oder verzweigte ($C_1$-$C_6$)-Alkylgruppen, geradkettige oder verzweigte ($C_1$-$C_6$)-Trihalogenoalkylgruppen, geradkettige oder verzweigte ($C_1$-$C_6$)-Alkoxygruppen, Hydroxygruppen, Nitrogruppen, Cyanogruppen oder Aminogruppen (die gegebenenfalls durch eine oder mehrere geradkettige oder verzweigte ($C_1$-$C_6$)-Alkylgruppen substituiert sind) zu verstehen ist,
- unter einem gegebenenfalls substituierten Heterocyclus eine mono- oder bicyclische, gesättigte oder ungesättigte Gruppe mit 5 bis 16 Kettengliedern zu verstehen ist, die ein, zwei oder drei Heteroatome ausgewählt aus Sauerstoff, Stickstoff oder Schwefel enthält, wobei der Heterocyclus gegebenenfalls durch ein oder mehrere Halogenatome, geradkettige oder verzweigte ($C_1$-$C_6$)-Alkylgruppen, geradkettige oder verzweigte ($C_1$-$C_6$)-Alkoxygruppen, Hydroxygruppen, Trihalogenomethylgruppen, Nitrogruppen, Cyanogruppen oder Aminogruppen (die gegebenenfalls durch eine oder mehrere geradkettige oder verzweigte ($C_1$-$C_6$)-Alkylgruppen substituiert sein können) substituiert sein kann.

**2.** Verbindungen der Formel (I) nach Anspruch 1, worin A zusammen mit den beiden benachbarten Kohlenstoffatomen des Phenylrings einen gegebenenfalls substituierten Naphthylring bildet.

**3.** Verbindungen der Formel (I) nach Anspruch 1, worin A zusammen mit den beiden benachbarten Kohlenstoffatomen des Phenylrings einen gegebenenfalls substituierten Heterocyclus ausgewählt aus gegebenenfalls substituierten Indolringen, gegebenenfalls substituierten Benzo[b]thiophenringen und gegebenenfalls substituierten Benzo[b]furanringen bedeutet.

**4.** Verbindungen der Formel (I) nach Anspruch 1, worin Z eine $C_4$-$C_{12}$-Alkylenkette darstellt, in der 1, 2 oder 3 Gruppen $-CH_2-$ durch 1, 2 oder 3 Gruppen -NR- ersetzt sind (worin R ein Wasserstoffatom oder eine geradkettige oder verzweigte ($C_1C_6$)-Alkylgruppe darstellt).

**5.** Verbindungen der Formel (I) nach Anspruch 1, worin

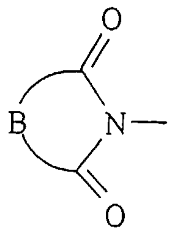

eine Gruppe

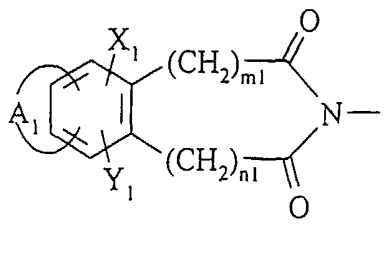

darstellt, in der:

| | |
|---|---|
| $m_1$ und $n_1$, | die gleichartig oder verschieden sind, 0 oder 1 darstellen, |
| $X_1$ und $Y_1$, | die gleichartig oder verschieden sind, ein Wasserstoffatom, ein Halogenatom, eine geradkettige oder verzweigte ($C_1$-$C_6$)-Alkylgruppe, eine geradkettige oder verzweigte ($C_1$-$C_6$)-Trihalogenoalkylgruppe, eine geradkettige oder verzweigte ($C_1$-$C_6$)-Alkoxygruppe, eine Hydroxygruppe, eine Cyanogruppe, eine Nitrogruppe, eine Aminogruppe, eine Alkylaminogruppe oder eine Dialkylaminogruppe darstellen und |
| $A_1$ | zusammen mit den beiden benachbarten Kohlenstoffatomen des Phenylrings: |

- einen gegebenenfalls substituierten Phenylring,
- einen gegebenenfalls substituierten Naphthylring,
- einen gegebenenfalls substituierten Tetrahydronaphthylring oder einen gegebenenfalls substituierten 1,4-Dioxo-1,2,3,4-tetrahydronaphthylring oder
- einen gegebenenfalls substituierten Heterocyclus bildet,

deren Stereoisomere sowie deren Additionssalze mit einer pharmazeutisch annehmbaren Säure oder Base.

**6.** Verbindungen der Formel (I) nach Anspruch 1, worin

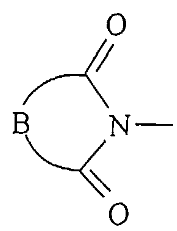

die Gruppe

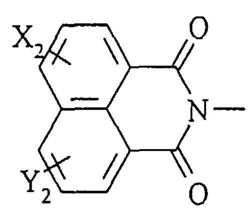

darstellt,
in der $X_2$ und $Y_2$, die gleichartig oder verschieden sind, ein Wasserstoffatom, ein Halogenatom, eine geradkettige oder verzweigte ($C_1$-$C_6$)-Alkylgruppe, eine geradkettige oder verzweigte ($C_1$-$C_6$)-Trihalogenoalkylgruppe, eine geradkettige oder verzweigte ($C_1$-$C_6$)-Alkoxygruppe, eine Hydroxygruppe, eine Cyanogruppe, eine Nitrogruppe, eine Aminogruppe, eine Alkylaminogruppe oder eine Dialkylaminogruppe bedeuten,
deren Stereoisomere sowie deren Additionssalze mit einer pharmazeutisch annehmbaren Säure oder Base.

**7.** Verbindung der Formel (I) nach Anspruch 1, in der m und n gleichzeitig 0 bedeuten.

**8.** Verbindung der Formel (I) nach Anspruch 1, nämlich N,N'-Bis[2-(2-aza-1,3-dioxo-10-methoxy-cyclopenta[c]phenanthren-2-yl)-ethyl]-ethan-1,2-diamin sowie dessen Additionssalze mit einer pharmazeutisch annehmbaren Säure.

**9.** Verbindung der Formel (I) nach Anspruch 1, nämlich N,N'-Bis[2-(2-aza-1,3-dioxo-10-methoxy-cyclopenta[c]phenanthren-2-yl)-ethyl]-propan-1,3-diamin sowie dessen Additionssalze mit einer pharmazeutisch annehmbaren Säure.

**10.** Verbindung der Formel (I) nach Anspruch 1, nämlich 1-[2-(1,3-Dioxo-2,3-dihydro-5-nitro-benzo[d,e]isochinolin-2-yl)-ethyl]-3-[2-(2-aza-1,3-dioxo-10-methoxy-cyclopenta[c]phenanthren-2-yl)-ethyl]-propan-1,3-diamin sowie dessen Additionssalze mit einer pharmazeutisch annehmbaren Säure.

**11.** Verbindung der Formel (I) nach Anspruch 1, nämlich 1-[2-(1,3-Dioxo-2,3-dihydro-5-nitro-benzo[d,e]isochinolin-2-yl)-ethyl]-2-[2-(2-aza-1,3-dioxo-10-methoxy-cyclopenta[c]phenanthren-2-yl)-ethyl]-ethan-1,2-diamin sowie dessen Additionssalze mit einer pharmazeutisch annehmbaren Säure.

**12.** Verfahren zur Herstellung der Verbindungen der Formel (I) nach Anspruch 1, dadurch gekennzeichnet, daß man als Ausgangsprodukt ein Anhydrid der Formel (II) verwendet:

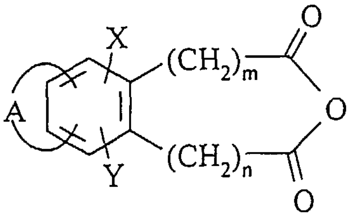

(II)

in der A, X, Y, m und n die bezüglich der Formel (I) angegebenen Bedeutungen besitzen, welches man mit einem Überschuß eines Diamins der Formel (III) umsetzt:

$$H_2N - Z - NH_2 \qquad (III)$$

in der Z die bezüglich der Formel (I) angegebenen Bedeutungen besitzt, so daß man nach der Trennung:

- entweder die Verbindung der Formel (I/a), einem Sonderfall der Verbindungen der Formel (I) erhält:

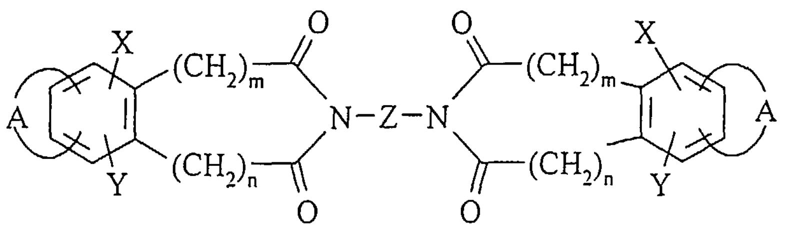

(I/a)

in der A, X, Y, Z, m und n die bezüglich der Formel (I) angegebenen Bedeutungen besitzen,

- oder die Verbindung der Formel (IV) erhält:

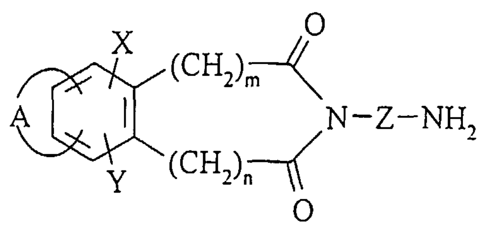

(IV)

in der A, X, Y, Z, m und n die bezüglich der Formel (I) angegebenen Bedeutungen besitzen,

- welche man:

  * <u>entweder</u> mit dem Anhydrid der Formel (II/a) umsetzt:

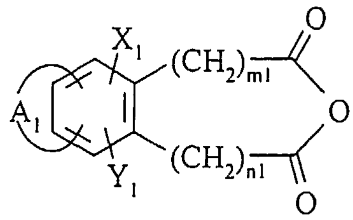

(II/a)

in der $A_1$, $X_1$, $Y_1$, $m_1$ und $n_1$ die bezüglich der Formel (I) angegebenen Bedeutungen besitzen, zur Bildung der Verbindung der Formel (I/b), einem Sonderfall der Verbindungen der Formel (I):

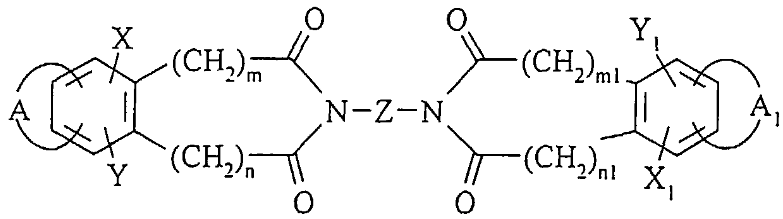

(I/b)

in der A, X, Y, m, n, Z, $A_1$, $X_1$, $Y_1$, $m_1$ und $n_1$ die bezüglich der Formel (I) angegebenen Bedeutungen besitzen,

* <u>oder</u> mit dem Anhydrid der Formel (V) umsetzt:

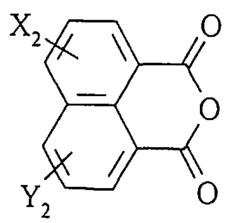

(V)

in der $X_2$ und $Y_2$ die bezüglich der Formel (I) angegebenen Bedeutungen besitzen,
zur Bildung der Verbindung der Formel (I/c), einem Sonderfall der Verbindungen der Formel (I):

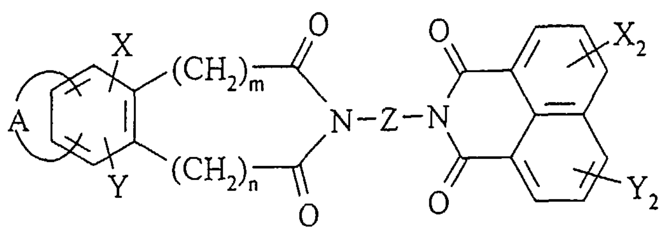

(I/c)

in der A, X, Y, $X_2$, $Y_2$, Z, m und n die bezüglich der Formel (I) angegebenen Bedeutungen besitzen,

welche Verbindung der Formel (I/a), (I/b) oder (I/c) man gewünschtenfalls klassischen Reaktionen zur Umwandlung der Substituenten an den aromatischen Kernen unterwerfen kann, welche man gegebenenfalls mit Hilfe einer klassischen Reinigungsmethode reinigen kann, gegebenenfalls mit Hilfe einer klassischen Methode in ihre Stereoisomeren auftrennen kann und gewünschtenfalls in ihre Additionssalze mit einer pharmazeutisch annehmbaren Säure oder Base umwandeln kann.

**13.** Pharmazeutische Zubereitungen enthaltend als Wirkstoff mindestens eine Verbindung nach einem der Ansprüche 1 bis 11 allein oder in Kombination mit einem oder mehreren inerten, nichttoxischen, pharmazeutisch annehmbaren Trägermaterialien oder Bindemitteln.

**14.** Pharmazeutische Zubereitungen nach Anspruch 13, enthaltend mindestens einen Wirkstoff nach einem der Ansprüche 1 bis 11 als Antikrebsmittel.

## Claims

**1.** Compounds of formula (I):

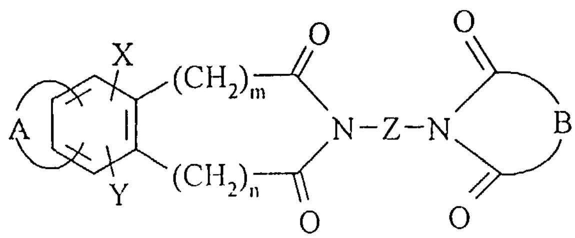

(I)

wherein:

m and n, which may be the same or different, represent 0 or 1,

X and Y, which may be the same or different, represent a hydrogen or halogen atom or a linear or branched $(C_1\text{-}C_6)$alkyl, linear or branched $(C_1\text{-}C_6)$trihaloalkyl, linear or branched $(C_1\text{-}C_6)$alkoxy, hydroxy, cyano, nitro, amino, alkylamino or dialkylamino group,

Z represents a linear or branched $C_4$ to $C_{12}$ alkylene chain in which one or more $-CH_2-$ groups are optionally replaced by any of the atoms or groups: -NR- (wherein R represents a hydrogen atom or a linear or branched $(C_1\text{-}C_6)$alkyl group), -O-, -S-, -SO-, $-SO_2-$, -CONH- or by a substituted or unsubstituted heterocyclic group,

A, together with two adjacent carbon atoms of the phenyl ring, forms :

- a substituted or unsubstituted phenyl ring,
- a substituted or unsubstituted naphthyl ring system,
- a substituted or unsubstituted tetrahydronaphthyl ring system or a substituted or unsubstituted 1,4-dioxo-1,2,3,4-tetrahydronaphthyl ring system,
- or a substituted or unsubstituted heterocycle,

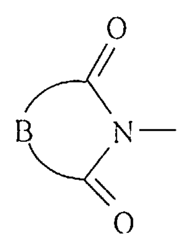

represents any of the following groups :

*

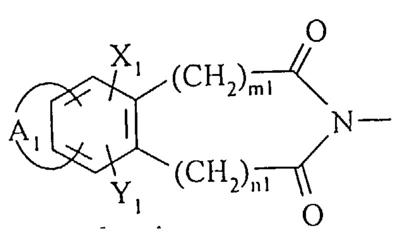

wherein:

$m_1$ and $n_1$, which may be the same or different, represent 0 or 1,

$X_1$ and $Y_1$, which may be the same or different, represent a hydrogen or halogen atom or a linear or branched ($C_1$-$C_6$)alkyl, linear or branched ($C_1$-$C_6$)trihaloalkyl, linear or branched ($C_1$-$C_6$)alkoxy, hydroxy, cyano, nitro, amino, alkylamino or dialkylamino group,

$A_1$, together with two adjacent carbon atoms of the phenyl ring, forms :

- a substituted or unsubstituted phenyl ring,
- a substituted or unsubstituted naphthyl ring system,
- a substituted or unsubstituted tetrahydronaphthyl ring system or a substituted or unsubstituted 1,4-dioxo-1,2,3,4-tetrahydronaphthyl ring system,
- or a substituted or unsubstituted heterocycle,

*

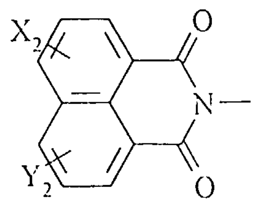

wherein $X_2$ and $Y_2$, which may be the same or different, represent a hydrogen or halogen atom or a linear or branched ($C_1$-$C_6$)alkyl, linear or branched ($C_1$-$C_6$)trihaloalkyl, linear or branched ($C_1$-$C_6$)alkoxy, hydroxy, cyano, nitro, amino, alkylamino or dialkylamino group,

their stereoisomers, and addition salts thereof with a pharmaceutically acceptable acid or base,
it being understood that:

- a substituted or unsubstituted phenyl group, a substituted or unsubstituted naphthyl group or a substituted or unsubstituted tetrahydronaphthyl group is understood to be a phenyl, naphthyl or tetrahydronaphthyl group optionally substituted by one or more halogen atoms or groups linear or branched ($C_1$-$C_6$)alkyl, linear or branched ($C_1$-$C_6$)trihaloalkyl, linear or branched ($C_1$-$C_6$)alkoxy, hydroxy, nitro, cyano or amino (optionally substituted by one or more linear or branched ($C_1$-$C_6$)alkyl groups),

- a substituted or unsubstituted heterocycle is understood to be a saturated or unsaturated, mono- or bi-cyclic, 5- to 16-membered group containing one, two or three hetero atoms selected from oxygen, nitrogen and sulphur, it being understood that the heterocycle may be optionally substituted by one or more halogen atoms or groups linear or branched ($C_1$-$C_6$)alkyl, linear or branched ($C_1$-$C_6$)alkoxy, hydroxy, trihalomethyl, nitro, cyano or amino (optionally substituted by one or more linear or branched ($C_1$-$C_6$)alkyl groups).

**2.** Compounds of formula (I) according to claim 1, in which A, together with two adjacent carbon atoms of the phenyl ring, represents a substituted or unsubstituted naphthyl ring system.

**3.** Compounds of formula (I) according to claim 1, in which A, together with two adjacent carbon atoms of the phenyl ring, represents a substituted or unsubstituted heterocycle selected from optionally substituted indole, optionally substituted benzo[b]thiophene and optionally substituted benzo[b]furan ring systems.

**4.** Compounds of formula (I) according to claim 1, in which Z represents a $C_4$ to $C_{12}$ alkylene chain in which 1, 2 or 3 $-CH_2-$ groups are replaced by 1, 2 or 3 -NR- groups (wherein R represents a hydrogen atom or a linear or branched ($C_1$-$C_6$)alkyl group).

**5.** Compounds of formula (I) according to claim 1, in which

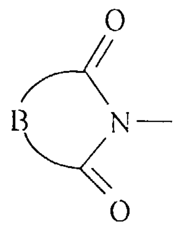

represents the group

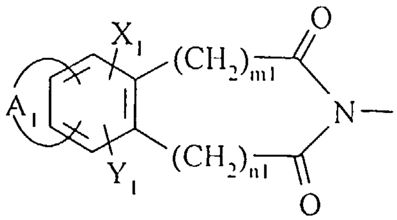

wherein :

$m_1$ and $n_1$, which may be the same or different, represent 0 or 1,

$X_1$ and $Y_1$, which may be the same or different, represent a hydrogen or halogen atom, a linear or branched $(C_1-C_6)$alkyl, linear or branched $(C_1-C_6)$trihaloalkyl, linear or branched $(C_1-C_6)$alkoxy, hydroxy, cyano, nitro, amino, alkylamino or dialkylamino group,

$A_1$, together with two adjacent carbon atoms of the phenyl ring, forms :

- a substituted or unsubstituted phenyl ring,
- a substituted or unsubstituted naphthyl ring system,
- a substituted or unsubstituted tetrahydronaphthyl ring system or a substituted or unsubstituted 1,4-dioxo-1,2,3,4-tetrahydronaphthyl ring system,
- or a substituted or unsubstituted heterocycle,

their stereoisomers, and addition salts thereof with a pharmaceutically acceptable acid or base.

**6.** Compounds of formula (I) according to claim 1, in which

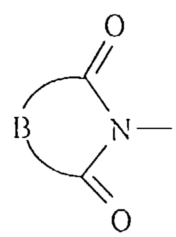

represents the group

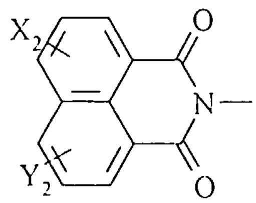

wherein $X_2$ and $Y_2$, which may be the same or different, represent a hydrogen or halogen atom or a linear or branched $(C_1-C_6)$alkyl, linear or branched $(C_1-C_6)$trihaloalkyl, linear or branched $(C_1-C_6)$alkoxy, hydroxy, cyano, nitro, amino, alkylamino or dialkylamino group,
their stereoisomers, and addition salts thereof with a pharmaceutically acceptable acid or base.

**7.** Compound of formula (I) according to claim 1, in which m and n simultaneously represent 0.

**8.** Compound of formula (I) according to claim 1 which is N,N'-bis[2-(2-aza-1,3-dioxo-10-methoxy-cyclopenta[c]phenanthren-2-yl)ethyl]ethane-1,2-diamine and addition salts thereof with a pharmaceutically acceptable acid.

**9.** Compound of formula (I) according to claim 1 which is N,N'-bis[2-(2-aza-1,3-dioxo-10-methoxy-cyclopenta[c]phenanthren-2-yl)ethyl]propane-1,3-diamine and addition salts thereof with a pharmaceutically acceptable acid.

**10.** Compound of formula (I) according to claim 1 which is 1-[2-(1,3-dioxo-2,3-dihydro-5-nitro-benzo[d,e]isoquinolin-2-yl)ethyl]-3-[2-(2-aza-1,3-dioxo-10-methoxy-cyclopenta[c]phenanthren-2-yl)ethyl]propane-1,3-diamine and addition salts thereof with a pharmaceutically acceptable acid.

**11.** Compound of formula (I) according to claim 1 which is 1-[2-(1,3-dioxo-2,3-dihydro-5-nitro-benzo[d,e]isoquinolin-2-yl)ethyl]-2-[2-(2-aza-1,3-dioxo-10-methoxy-cyclopenta[c]phenanthren-2-yl)ethyl]ethane-1,2-diamine and addition salts thereof with a pharmaceutically acceptable acid.

**12.** Process for the preparation of compounds of formula (I) according to claim 1, characterised in that there is used as starting material an anhydride of formula (II) :

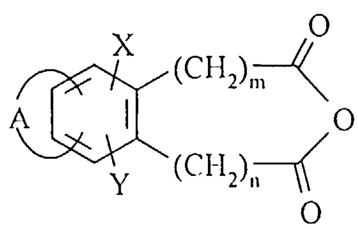

(II)

wherein A, X, Y, m and n are as defined for formula (I),
which is reacted with an excess of a diamine of formula (III):

$$H_2N - Z - NH_2 \qquad (III)$$

wherein Z is as defined for formula (I),
to yield after separation :

- either the compound of formula (I/a), a particular case of the compounds of formula (I) :

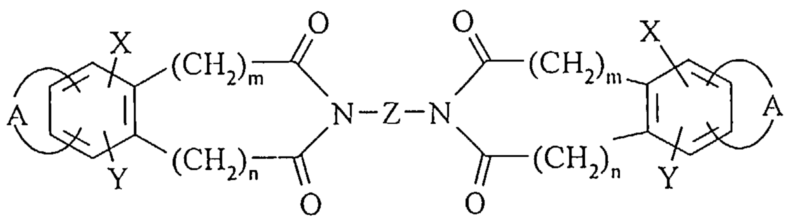

(I/a)

wherein A, X, Y, Z, m and n are as defined for formula (I),

- or the compound of formula (IV):

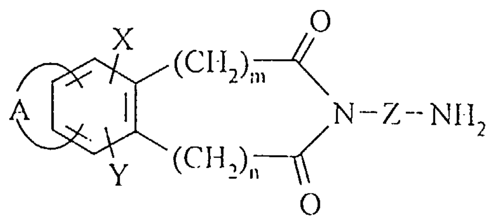

(IV)

wherein A, X, Y, Z, m and n are as defined for formula (I),

- which is reacted :

  * *either* with the anhydride of formula (II/a) :

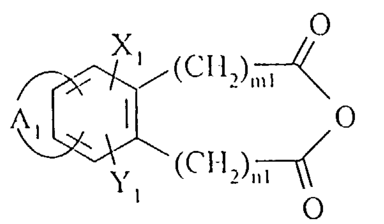

(II/a)

wherein $A_1$, $X_1$, $Y_1$, $m_1$ and $n_1$ are as defined for formula (I), to yield the compound of formula (I/b), a particular case of the compounds of formula (I) :

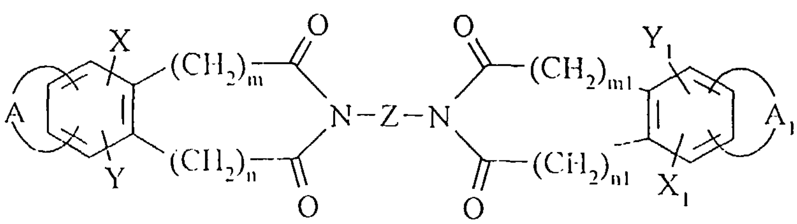

(I/b)

wherein A, X, Y, m, n, Z, $A_1$, $X_1$, $Y_1$, $m_1$ and $n_1$ are as defined for formula (I):

  * *or* with the anhydride of formula (V):

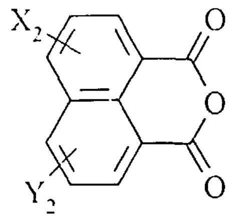

(V)

wherein $X_2$ and $Y_2$ are as defined for formula (I),

to yield the compound of formula (I/c), a particular case of the compounds of formula (I):

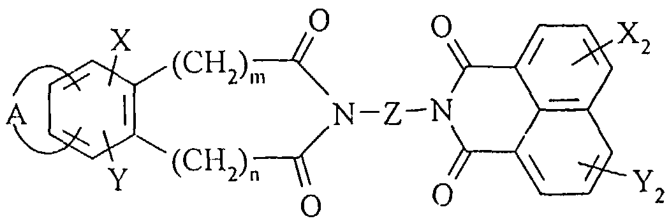

(I/c)

wherein A, X, Y, $X_2$,$Y_2$, Z, m and n are as defined for formula (I),

which compound of formula (I/a), (I/b) or (I/c) may be subjected, if desired, to the conventional conversion reactions of substituents on aromatic nuclei, is purified, if necessary, according to a conventional purification technique, is separated, where appropriate, into its stereoisomers according to a conventional separation technique, and which is converted, if desired, into its addition salts with a pharmaceutically acceptable acid or base.

**13.** Pharmaceutical compositions comprising, as active ingredient, at least one compound according to any one of claims 1 to 11, alone or in combination with one or more pharmaceutically acceptable, inert, non-toxic excipients or carriers.

**14.** Pharmaceutical compositions according to claim 13 comprising at least one active ingredient according to any one of claims 1 to 11 for use as anticancer agents.